# EUROPÄISCHE PATENTSCHRIFT

(11) **EP 3 183 257 B1**
(45) Veröffentlichungstag und Bekanntmachung des Hinweises auf die Patenterteilung: **09.10.2019**
(21) Anmeldenummer: 15774500.1
(22) Anmeldetag: 17.08.2015
(51) Int. Cl.: C07F 9/6558, A61K 31/66, A61K 31/70

(54) **DI- UND TRIPHOSPHAT-PROPHARMAKA**
DI- AND TRIPHOSPHATE PRODRUGS
PROMÉDICAMENTS À BASE DE DIPHOSPHATE ET DE TRIPHOSPHATE

(30) Priorität: 22.08.2014 DE 102014112055
(43) Veröffentlichungstag der Anmeldung: 28.06.2017
(73) Patentinhaber: Universität Hamburg, 20148 Hamburg (DE)
(72) Erfinder: MEIER, Chris, 21635 Jork (DE); GOLLNEST, Tristan, 22926 Ahrensburg (DE); NACK, Tobias, 22303 Hamburg (DE); WEINSCHENK, Lina, 21107 Hamburg (DE)
(74) Vertreter: Stüven, Ralf
(86) Internationale Anmeldenummer: PCT/DE2015/200440
(87) Internationale Veröffentlichungsnummer: WO 2016/026493

(56) Entgegenhaltungen:
- WO-A2-2009/129798

## Beschreibung

Die Erfindung betrifft als Propharmaka einsetzbare Verbindungen, insbesondere Nukleosiddi- und -triphosphat-Propharmaka, und ein Verfahren zur Herstellung dieser Verbindungen.

Bei der Behandlung von zum Beispiel viralen Infektionskrankheiten wie Herpes- oder Hepatitis-Infektionen oder der Immunschwächekrankheit AIDS (Acquired Immunodeficiency Syndrome), aber auch bei Krebsleiden, werden Nukleosidanaloga eingesetzt, welche in DNA eingebaut werden können. Nach dem Einbau wirken die Nukleosidanaloga häufig als Kettenterminatoren, d.h. es findet keine weitere Elongation in 3'-Richtung statt (J. Balzarini, P. Herdewijn, E. De Clercq; Differential Patterns of intracellular Metabolism of 2',3'-Didehydro-2',3'-dideoxythymidine and 3'-Azido-2',3'-dideoxythymidine, two potent Anti-human Immunodeficiency Virus Compounds; J. Biol. Chem. 1989, 264, 6127-6133). Die Nukleosidanaloga müssen jedoch als Triphosphate (NTP) vorliegen, damit sie zur Kettenverlängerung in den DNA-Strang eingebaut werden können.

In Form ihrer Mono-, Di- oder Triphosphate (NMP, NDP, NTP) können Nukleosidanaloga die Zellmembran aufgrund ihrer Ladung unter physiologischen Bedingungen nicht durchdringen. Auch ein Passieren der Blut-Hirn-Schranke (BBB; blood brain barrier) zur Behandlung von Erkrankungen, die auch das Gehirn betreffen, ist nicht möglich. Nukleosidanaloga müssen daher entweder in der Zelle zunächst durch bestimmte, mehr oder minder spezifische Kinasen in mehreren Stufen in ihre Triphosphate überführt werden, oder die Nukleosidanaloga müssen so ausgestaltet oder modifiziert werden, dass sie in der Lage sind, die Zellmembran bzw. BBB zu passieren.

Hierzu muss in der Regel die Lipophilie der Medikamente erhöht werden (z.B. R. J. Sawchuk, Z. Yang; Investigation of distribution, transport and uptake of anti-HIV drugs to the central nervous system; Advanced Drug Delivery Reviews 1999, 39, 5-31). Eine Möglichkeit, die Lipophilie bekannter Medikamente zu erhöhen, liegt in der Verwendung von Propharmaka-Systemen. Propharmaka oder "Prodrugs" sind Wirkstoffvorläufer, welche den eigentlichen Wirkstoff idealerweise erst am gewünschten Wirkort unter Abspaltung von Maskierungsgruppen freisetzen. Neben der erhöhten Lipophilie sind weitere Voraussetzungen für solche Propharmaka eine ausreichende Stabilität im extrazellulären Medium und die Freisetzung nicht toxischer Masken.

Enzymatisch aktivierbare Prodrug-Systeme für Nukleosidmonophosphate (NMP) sind bereits beschrieben worden (A. Pompon, I. Lefebvre, J.-L. Imbach, S. Khan, D. Farquhar; Decomposition Pathways of the Mono-(Pivaloyloxymethyl) and Bis-(Pivaloyloxymethyl) Esters of Azidothymidine-5'-Monophosphate in Cell Extract and in Tissue-Culture Medium - An Application of the Online Isrp-Cleaning HPLC Technique; Antiviral Chem. Chemother. 1994, 5, 91-98; I. Lefebvre, C. Perigaud, A. Pompon, A.-M. Aubertin, J.-L. Girardet, A. Kim, G. Gosselin, J.-L. Imbach; MonoNukleoside Phosphotriester Derivates with S Acyl-2-thioethyl Bioreversible Phosphate-Protecting Groups: Intracellular Delivery of 3'-Azido-2',3'-dideoxythymidine-5'-monophosphate; J. Med. Chem. 1995, 38, 3941-3950; W. Thomson, D. Nicholls, W. J. Irwin, J. S. Al-Mushadani, S. Freeman, A. Karpas, J.Petrik, N. Mahmood, A. J. Hay, Synthesis, Bioactivation and Anti-HIV Activity of the Bis(4-acyloxybenzyl) and Mono(4-acyloxybenzyl) Esters of the 5'-Monophosphate of AZT, J. Chem. Soc., Perkin Trans., 1993, 1, 1239-1245; Thomson, D. Nicholls, W. J. Irwin, J. S. Al-Mushadani, S. Freeman, A. Karpas, J.Petrik, N. Mahmood, A. J. Hay, Synthesis, Bioactivation and Anti-HIV Activity of the Bis(4-acyloxybenzyl) and Mono(4-acyloxybenzyl) Esters of the 5'-Monophosphate of AZT, J. Chem. Soc., Perkin Trans., 1993, 1, 1239-1245; A. Routledge, I. Walker, S. Freeman, A. Hay, N. Mahmood, Synthesis, Bioactivation and Anti-HIV Activity of 4-Acyloxybenzyl Bis(Nucleosid-5-yl) Phosphates; Nucl. Nucl., 1995, 14, 1545-1558; C. Meier, CycloSal Phosphates as Chemical trojan Horses for the intracellular Nucleotide and Glycosylmonophosphate Delivery - Chemistry meets Biology; European Journal of Organic Chemistry 2006, 1081-1102). Nachteilig am Einsatz von NMP-Prodrugs ist allerdings, dass die erforderliche weitere Phosphorylierung zu den Di- und Triphosphaten in der Zelle gehemmt oder vollständig unterbunden sein kann. So ist beispielsweise im Fall des Nukleosid-Analogons Azidothymidin (AZT), einem bekannten Anti-HIV-Medikament, die Phosphorylierung zum AZTDP (DP = Diphosphat) gehemmt. Darüber hinaus werden zahlreiche Nebenwirkungen dem entsprechenden Monophosphat AZTMP (MP = Monophosphat) zugeschrieben.

Für die Maskierung von Nukleosiddiphosphaten (NDP) und Nukleosidtriphosphaten (NTP) gibt es nach wie vor keine zufrieden stellende Lösung. Problematisch ist hierbei, dass im Gegensatz zu NMP nicht nur eine Phosphatgruppe vorliegt, sondern eine bzw. zwei energiereiche Phosphorsäureanhydridbindung(en), die in Form der Pyrophosphateinheit reversibel maskiert werden muss(müssen), ohne dass es zu einem Bruch der Anhydrid-Bindung(en) kommt. Bei der Demaskierung der Pyrophosphateinheit darf keine Reaktion am Phosphoratom stattfinden, da dies zu einem Bruch der Pyrophosphatbrücke führen kann. Dies unterscheidet NDP- und NTP-Prodrugs essentiell von ihren NMP-Verwandten. Hier können auch Hydrolysereaktionen am Phosphoratom stattfinden.

In der WO 2009/129798 A2 sind symmetrisch maskierte Nukleosiddi- und -triphosphate sowie ein Verfahren zu deren Herstellung beschrieben. "Symmetrisch" bedeutet in diesem Zusammenhang, dass die verwendeten Masken zur Maskierung der Ladungen des endständigen Phosphats dieselbe Struktur aufweisen. Es hat sich jedoch herausgestellt, dass der bei der chemischen oder enzymatischen Hydrolyse freigesetzte prozentuale Anteil an der gewünschten Diphosphat- bzw. Triphosphatspezies des Wirkstoffes von der Geschwindigkeit der Abspaltung der ersten Maske unter der Freisetzung des monomaskierten Intermediats abhängt. Als Nebenprodukt wurden die entsprechenden Hydrolyseprodukte der Pyrophosphatgruppe detektiert, im Fall der Nukleosiddiphosphat-Prodrugs die entsprechenden Nukleosidmonophosphate, bei den Nukleosidtriphosphaten die entsprechenden Nukleosiddiphosphate und die Nukleosidmonophosphate. Daraus resultiert letztlich eine nur unselektive Freisetzung der gewünschten Zielverbindungen.

Jessen et al. 2008 und Schulz et al. 2014 (H.J. Jessen, T. Schulz, J. Balzarini, C. Meier, Bioreversible protection of nucleosidediphosphates; Angewandte Chemie International Edition English 2008, 47, 8719-8722; T. Schulz, J. Balzarini, C. Meier, The DiPPro Approach: Synthesis, Hydrolysis, and Antiviral Activity of Lipophilic d4T Diphosphate Prodrugs; ChemMedChem 2014, 9, 762-75; s. auch Schulz, T., 2012, Synthese und Untersuchung von Nucleosiddiphosphat Prodrugs, Dissertation, Universität Hamburg) beschreiben ebenfalls symmetrisch maskierte Nukleosiddiphosphate. Darüber hinaus ist in Schulz et al. 2014 auch die asymmetrische Maskierung eines Nukleosiddiphosphatanalogons (1-[(2R,5S)-5-(Hydroxymethyl)-2,5-dihydrofuran-2-yl]-5-methylpyrimidin-2,4-dion, Stavudin, d4T) mit einer Acyloxybenzylgruppe und einer β-Cyanoethylgruppe beschrieben. Die β-Cyanoethyl-Maske ist jedoch nur chemisch und nicht enzymatisch abspaltbar, was jedoch für einen Einsatz als Arzneimittel erforderlich wäre.

Aufgabe der vorliegenden Erfindung ist es daher, weiter verbesserte Di- und/oder Triphosphat-Propharmaka, insbesondere Nukleotid- bzw. Nukleotidanalogon-Propharmaka, bereitzustellen, die die aus dem Stand der Technik bekannten Nachteile nicht aufweisen. Überraschenderweise gelingt die Lösung dieses Problems durch eine geeignete bioreversible asymmetrische Maskierung von Di- und Triphosphatverbindungen, insbesondere von Nukleosiddiphosphaten und Nukleosidtriphosphaten bzw. deren Analoga, wobei die Maskierung nur am endständigen, d.h. am β- bzw. γ-Phosphat durchgeführt wird, während auf die Maskierung des innenständigen Phosphats verzichtet wird. Mit der Erfindung wird eine signifikant verbesserte bioreversible Maskierung von Nukleosiddiphosphaten (NDP) und Nukleosidtriphosphaten (NTP) sowie deren Analoga erreicht.

Zur Lösung der Aufgabe stellt die vorliegende Erfindung in einem ersten Aspekt eine Verbindung der allgemeinen Formel I 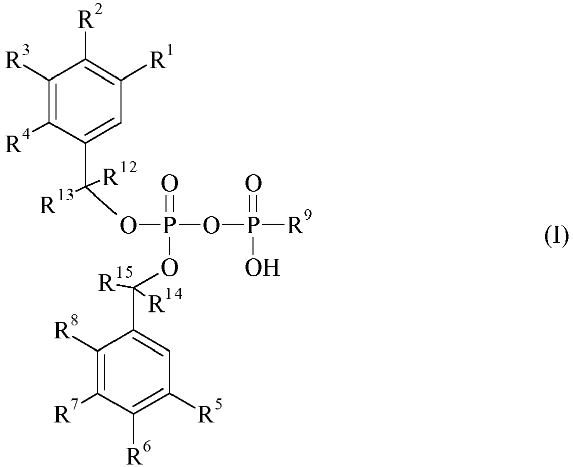 oder ein pharmazeutisch annehmbares Salz davon bereit, wobei
R¹, R³, R⁵ und R⁷ unabhängig voneinander H, Halogen, NO₂, CN, SO₃H, ein cyclischer, acyclischer, linearer oder verzweigter aliphatischer oder
heteroaliphatischer Rest, oder ein aromatischer oder heteroaromatischer Rest sind,
R² und R⁴ unabhängig voneinander H oder Z-C(Y)-R_{A}, jedoch nicht beide H sind,
R⁶ und R⁸ unabhängig voneinander H, Z-C(Y)-R_{B}, jedoch nicht beide H sind,
Z, Y unabhängig voneinander O, S oder HN ist,
R_{A} und R_{B} verschieden und jeweils ein cyclischer, acyclischer, linearer oder verzweigter aliphatischer oder heteroaliphatischer Rest, oder ein aromatischer oder heteroaromatischer Rest sind,
R⁹ Nukleosid, Nukleosidmonophosphat, Nukleosidanalogon, Nukleosidmonophosphatanalogon, O-R¹⁰, OP(O)(OH)-R¹⁰ oder OP(O)(OH)-O-R¹⁰ ist, wobei
R¹⁰ ein cyclischer, acyclischer, linearer oder verzweigter aliphatischer oder heteroaliphatischer Rest, oder ein aromatischer oder heteroaromatischer Rest ist,
R¹², R¹³, R¹⁴ und R¹⁵ unabhängig voneinander H, ein cyclischer, acyclischer, linearer oder verzweigter aliphatischer oder heteroaliphatischer Rest, ein aromatischer oder heteroaromatischer Rest, und/oder ein Elektronenakzeptor sind.

Bei den erfindungsgemäßen Verbindungen unterscheiden sich die Benzylderivat-Masken A und B 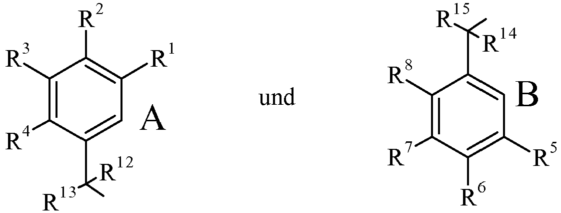 an der endständigen (terminalen) Phosphatgruppe aufgrund unterschiedlicher Reste R am Phenylring so voneinander, dass sie in der Zelle in selektiver Weise enzymatisch, z.B. mittels Esterasen, abgespalten werden können, um die gewünschte Diphosphat- oder Triphosphatverbindung freizusetzen. Um die gewünschte Selektivität zu erreichen, sind die Masken derart ausgestaltet, dass eine von ihnen instabiler ist, d.h. mit größerer Geschwindigkeit abgespalten wird und damit die schnelle Bildung eines monomaskierten Intermediats mit hoher Resistenz gegenüber einem nukleophilen Angriff auf die Phosphorsäureanhydridbindung(en) ermöglicht. Die zweite Gruppe ist lipophiler und sorgt damit für die notwendige Lipophilie, um die Aufnahme in die Zelle zu gewährleisten. Eine größere Abspaltungsgeschwindigkeit kann beispielsweise durch eine größere Hydrophilie (Polarität) der Maske bewirkt werden. Die Einstellung der Hydrophilie oder Lipophilie einer Maske ist dem Fachmann bekannt und kann gegebenenfalls durch Routineversuche ermittelt werden. Beispielsweise kann die Lipophilie einer Maske durch die Größe bzw. Länge eines Kohlenwasserstoffrestes beeinflusst werden, die Hydrophilie durch Vorsehen polarer Reste. Auch durch den Einbau von Heteroatomen kann beispielsweise die Stabilität der Maske beeinflusst werden.

Die Erfinder haben festgestellt, dass die Geschwindigkeiten der Spaltungen der beiden Masken mit ihrer Lipophilie häufig eng verknüpft sind, und zwar dergestalt, dass die Hydrolysehalbwertszeit einer Maske mit zunehmender Lipophilie zunimmt. Ohne an eine Theorie gebunden sein zu wollen, wird angenommen, dass eine Erhöhung der Lipophilie hauptsächlich durch das Anknüpfen von längeren hydrophoben Seitenketten, beispielsweise Alkylketten, erreicht wird, die dann aber aufgrund sterischer Hinderung in der Regel entsprechend schlechter von einem Nucleophil oder Enzym angegriffen werden können. Es ist allerdings zu beachten, dass der beschriebene Zusammenhang zwischen Lipophilie und Stabilität (Hydrolysehalbwertszeit) einer Maske lediglich ein Anhaltspunkt für die Auswahl geeigneter Maskenkombinationen ist. Auch eine lipophilere Maske kann in einer bestimmten Kombination im Einzelfall die instabilere Maske bilden. Der Fachmann kann dies anhand von Routineversuchen feststellen.

Die erfindungsgemäßen Verbindungen ermöglichen die Einschleusung von Nukleotiden und Nukleotidanaloga sowie anderer organischer Verbindungen mit einer Hydroxylgruppe, z.B. von Zuckern oder Alkoholen, auf dem Niveau von Di- und Triphosphaten in die Zelle. Nukleosidtriphosphate können somit direkt in die Zelle eingeführt werden, sodass ansonsten erforderliche weitere intrazelluläre Phosphorylierungsschritte nicht mehr benötigt werden. Dadurch kann nicht nur die tatsächliche Verwendung der eingeschleusten Nukleoside durch die Zelle besser ermöglicht, sondern es können auch Nebenwirkungen vermieden werden, die beispielsweise durch Monophosphate hervorgerufen werden. Die erfindungsgemäßen Verbindungen können beispielsweise sowohl als antivirale als auch als antitumorale Mittel vorteilhafte Verwendung als Arzneimittel finden. Sie eignen sich besonders als Arzneimittel zur Behandlung von Infektionen durch Viren, insbesondere Retroviren wie HI-Viren, Influenza-, hämorrhagische Fieber- und Hepatitis-Viren. Die Verbindungen eignen sich aber auch für analytische Zwecke bei biochemischen Untersuchungen, da die phosphorylierten Metaboliten intrazellulär freigesetzt werden. Dadurch werden Untersuchungen ermöglicht, die bislang noch nicht durchgeführt werden konnten. Auch nicht-nukleosidische Alkohole können beispielsweise in Form ihrer Di- (z.B. Isoprenyldiphosphat) oder Triphosphate in die Zelle transportiert und dort freigesetzt werden.

Die erfindungsgemäßen Verbindungen liegen unter physiologischen Bedingungen regelmäßig als Salze vor. Im Folgenden sind daher beispielhaft eine Diphosphatverbindung Ia (links) und eine Triphosphatverbindung Ib (rechts) dargestellt, wobei Kat⁺ Kation, beispielsweise Ammonium, Triethylammonium oder Tetrabutylammonium, bedeutet und R¹ bis R⁸ und R¹² bis R¹⁵ wie oben definiert sind. Nucl steht hier für Nukleosid oder Nukleosidanalogon.

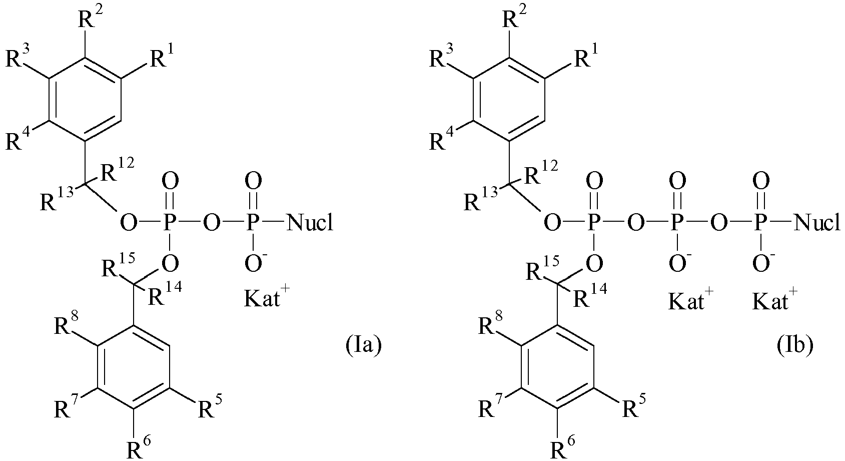

Unter einer "asymmetrisch maskierten Di- oder Triphosphatverbindung" wird eine Verbindung gemäß der folgenden Formel 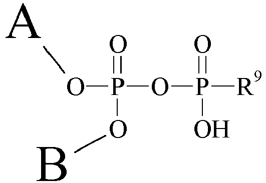 verstanden, wobei A und B für unterschiedliche chemische Strukturen stehen, und R⁹ wie oben definiert ist. Die terminale Phosphatgruppe bildet so ein stereogenes Zentrum. Die chemischen Strukturen A, B neutralisieren (maskieren) die negativen elektrischen Ladungen an den einfach gebundenen Sauerstoffatomen des endständigen Phosphats unter physiologischen Bedingungen. Die Masken selbst sind unter physiologischen Bedingungen ebenfalls nicht geladen. Unter einem endständigen Phosphat wird bei einer Diphosphatverbindung die β-, bei einer Triphosphatverbindung die γ-Phosphatgruppe verstanden. Insbesondere wird hier unter einer "asymmetrisch maskierten Di- oder Triphosphatverbindung" eine Verbindung verstanden, bei der die eine Maske, z.B. Maske A, instabiler ist und damit einer schnelleren enzymatischen Abspaltung unterliegt, während die andere Maske, z.B. Maske B, lipophiler ist.

Unter einer "enzymatisch abspaltbaren Maske" wird hier eine Maske verstanden, die mit Hilfe von in der Zielzelle vorhandenen oder gegebenenfalls induzierbaren Enzymen abspaltbar ist. Der Begriff umfasst auch solche Fälle, bei denen eine Maske in mehreren Schritten, beispielsweise kaskadenartig, abgebaut wird, und wenigstens ein Abbauschritt enzymatisch erfolgt. Beispielsweise kann der initiale Angriff enzymatisch erfolgen, während die weiteren Abbauschritte spontan erfolgen. Eine "chemisch abspaltbare Maske" ist demgegenüber eine Maske, die unter Bedingungen, wie sie in einer Zielzelle herrschen, beispielsweise hinsichtlich Temperatur, pH-Wert, Salzgehalt etc., nicht oder zumindest im Wesentlichen nicht oder mit zu geringer Geschwindigkeit abgespalten wird.

Unter einem "Nukleosid" (ggfs. auch "Nucleosid") werden hier organische Moleküle verstanden, die aus einem Zuckerrest (Zuckerkomponente) und einer organischen Base (Basenkomponente), z.B. einer heterocyclischen organischen Base, insbesondere einer stickstoffhaltigen heterocyclischen organischen Base (Nukleobase), bestehen, die über eine glykosidische Bindung verbunden sind. Der Zuckerrest ist häufig eine Pentose, z.B. Desoxyribose oder Ribose, kann aber auch ein anderer Zucker sein, z.B. ein C₃₋, C₄- oder C₆-Zucker. Häufig, aber nicht ausschließlich, sind Nukleobasen Purine (R) oder Pyrimidine (Y). Beispiele für natürlich vorkommende Purine sind Guanin (G) und Adenin (A), Beispiele für natürlich vorkommende Pyrimidine sind Cytosin (C), Thymin (T) und Uracil (U). Insbesondere wird unter einem Nukleosid daher eine Verbindung der allgemeinen Formel 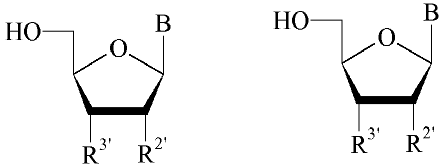 verstanden, wobei B eine stickstoffhaltige heterocyclische organische Base, z.B. eine Nukleobase, ist, und R^{2'} und R^{3'} unabhängig voneinander H oder OH sind. Phosphorylierte Nukleoside, beispielsweise Nukleosidmonophosphate (NMP), Nukleosiddiphosphate (NDP) und Nukleosidtriphosphate (NTP), werden auch als Nukleotide bezeichnet. Die Phosphat-, Diphosphat- (Pyrophosphat-) bzw. Triphosphatgruppe ist in der Regel mit dem 5'-C-Atom der Zuckerkomponente des Nukleosids verknüpft. In die Erfindung eingeschlossen sind aber auch Verbindung, bei denen die Phosphatgruppe(n) an eine 2'- oder 3'-OH-Gruppe gebunden sind.

Unter einem "Nukleosidanalogon" (ggfs. auch "Nucleosidanalogon") wird hier eine organische Verbindung verstanden, die im menschlichen Körper natürlicherweise nicht vorkommt, einem natürlicherweise im menschlichen Körper vorkommenden Nukleosid aber strukturell ähnlich ist, sodass es beispielsweise von der Zelle und/oder von viralen Enzymen im Wesentlichen entsprechend dem natürlichen Nukleosid verarbeitet, beispielsweise phosphoryliert und in einen RNA- oder DNA-Strang eingebaut werden kann. Ein Nukleosidanalogon kann selbst ein Nukleosid sein. Es kann aber beispielsweise auch eine andere Verbindung mit den obigen Eigenschaften sein, beispielsweise eine Verbindung aus einer heterocyclischen Base und einem acyclischen Rest und/oder einem Rest, der kein Zucker ist, oder eine Verbindung aus einer carbocyclischen Verbindung und einer heterocyclischen Base. Bei carbocyclischen Nucleosidanaloga ist beispielsweise der Ringsauerstoff in der Zuckerkomponente durch ein Kohlenstoff (eine Methylengruppe oder eine substituierte Methylengruppe) ersetzt. In der Zuckerkomponente kann beispielsweise auch ein C-Atom durch ein Heteroatom ersetzt sein, beispielsweise das 3'-C-Atom durch Schwefel. Nukleosidanaloga sind entweder selbst Nukleoside im obigen Sinne oder Nukleosiden strukturell und/oder funktionell analog. Da Nukleosidanaloga nicht notwendig eine Zucker- bzw. Basenkomponente im engeren Sinne enthalten müssen, wird hier ggfs. auch von einer zur Basenkomponente analogen Komponente (Basenanalogon) bzw. einer zur Zuckerkomponente analogen Komponente (Zuckeranalogon) gesprochen. Sofern hier von einer Zuckerkomponente oder Basenkomponente gesprochen wird, sollen die entsprechenden analogen Komponenten von Nukleosidanaloga mit erfasst sein, sofern sich aus dem Zusammenhang nicht eindeutig etwas anderes ergibt. Zahlreiche Nukleosidanaloga sind dem Fachmann bekannt. Bekannte Beispiele sind AZT (3'-Azido-2',3'-didesoxythimidin, Azidothymidin), 2',3'-Didesoxyinosin (Didanosin), 2',3'-Didesoxycytidin (Zalticabin), β-L-2',3'-Didesoxythiacytidine (Lamivudin, 3TC), L-Thymidine, 2'-Methyl-("up")-2'-hydroxyl-("down")-uridin/-cytidin, 2'-Methyl-("up")-2'-fluor-("down")-uridin/-cytidin (s. z.B. US 7608600 B1), 2-Amino-9-((2-hydroxyethoxy)methyl)-lH-purin-6(9H)-on (Acyclovir).

Unter einem "Nukleosidphosphatanalogon" wird hier ein Analogon zu einem phosphorylierten Nukleosid, d.h. ein Nukleotidanalogon, verstanden, unter einem "Nukleosidmonophosphatanalogon" somit ein Analogon zu einem Nukleosidmonophosphat. Beispiele für Nukleosidmonophosphatanaloga sind Nukleosidphosphonate wie beispielsweise 3-Hydroxy-2-phosphonomethoxypropyl (HPMP), 2-Phosphonomethoxyethyl (PME), 2',3'-didehydro-2',3'-dideoxythymidin-Phosphonat (d4TP), (S)-9-(3-Hydroxy-2-phosphonylmethoxypropyl)adenin (HPMPA) und 9-(2-Phosphonylmethoxyethyl)adenin (PMEA, Adefovir). Nukleosidphosphonate sind dem Fachmann bekannt, enthalten anstelle der P-O-Verknüpfung von Nukleosidphosphaten eine C-P-Verknüpfung, und können beispielsweise eine Nukleobase, eine acyclische oder cyclische aliphatische zuckeranaloge Komponente und eine Phosphonomethylgruppe CH₂P(O)(OH)₂-Gruppe enthalten (s. z.B. Pertusati et al. 2012, Medicinal Chemistry of Phosphonate Prodrugs for Antiviral Therapy, Antivir Chem Chemother. 22:181-203, doi: 10.3851/IMP2012). Auch phosphorylierte Nukleosidanaloga, beispielsweise phosphorylierte Nukleoside mit einer modifizierten Nukleobase, fallen unter die Begriffe "Nukleosidphosphatanalogon" oder "Nukleotidanalogon".

Sofern hier die Abkürzung "Nucl" verwendet wird, erfasst diese sowohl Nukleoside als auch Nukleosidanaloga. Die Abkürzungen "NMP", "NDP" und "NTP" erfassen, soweit nicht ausdrücklich etwas anderes angegeben ist, nicht nur Nukleosidmonophosphate, Nukleosiddiphosphate und Nukleosidtriphosphate, sondern auch entsprechende Analoga, also Nukleosidmonophosphatanaloga, Nukleosiddiphosphatanaloga und Nukleosidtriphosphatanaloga.

Bereichsangaben wie beispielsweise "1-10" sind hier immer so zu verstehen, dass jeder Zwischenwert mit offenbart ist. Im Fall einer Angabe, die nur Ganzzahlen betreffen kann, wie beispielsweise eine Zahl von C-Atomen, bedeutet dies selbstverständlich, dass nur Ganzzahlen offenbart sind. Auch ein beliebiger kleinerer Bereich aus dem Bereich soll dabei mit offenbart sein, wobei unter dem kleineren Bereich auch Bereiche zu verstehen sind, die keinen der Grenzwerte des Bereichs umfassen.

Der Ausdruck "Cₙ-Cₘ" oder "Cₙ₋ₘ", wobei n und m jeweils positive ganze Zahlen sind und m größer als n ist, bedeutet einen Bereich, der die Anzahl an C-Atomen einer Verbindung oder eines Restes angibt. Der Ausdruck soll hier ausdrücklich sämtliche ganzzahlige Zwischenwerte zwischen den Bereichsgrenzen n und m einschließen, und zwar jeweils unabhängig voneinander. Der Ausdruck "C₁₋₁₀" (n=1, m=10) bedeutet daher beispielsweise eine Verbindung, eine Gruppe oder einen Rest mit 1-10, d.h. 1, 2, 3, 4, 5, 6, 7, 8, 9 oder 10 C-Atomen. "C₁₋₁₀" umfasst daher gleichzeitig auch beispielsweise "C₂₋₆", d.h. 2, 3, 4, 5 oder 6 C-Atome, oder "C₁₋₄", d.h. 1, 2, 3 oder 4 C-Atome, oder "C₄₋₉", d.h. 4, 5, 6, 7, 8 oder 9 C-Atome. Entsprechend bedeutet der Begriff "C₁₋₂₀-Alkyl" beispielsweise eine Alkylgruppe mit 1, 2, 3, 4, 5, 6, 7, 8, 9, 10, 11, 12, 13, 14, 15, 16, 17, 18, 19 oder 20 C-Atomen und umfasst sämtliche Kombinationen aus den Werten von n und m, die in dem Bereich von n=1 bis m=20 liegen, z.B. _{"}C₁₋₁₀-Alkyl", d.h. ein Alkyl mit 1, 2, 3, 4, 5, 6, 7, 8, 9 oder 10 C-Atomen, oder "C₅₋₇-Alkyl", d.h. ein Alkyl mit 5, 6 oder 7 C-Atomen. Entsprechend verhält es sich auch mit Begriffen wie "C₂₋₁₀-Alkenyl", "C₄₋₂₀-Alkeninyl" und dergleichen.

Der Begriff "aliphatischer Rest" oder "Aliphatrest" umfasst cyclische oder acyclische, lineare (geradkettige) oder verzweigte, gesättigte oder ungesättigte Kohlenstoffverbindungsreste, außer aromatischen Resten. Der Begriff "heteroaliphatischer Rest" bedeutet aliphatische Reste, in deren Kohlenstoffgerüst ein oder mehrere C-Atome durch Heteroatome, beispielsweise Sauerstoff, Schwefel, Stickstoff oder Phosphor ersetzt sind.

Der Begriff "Alkyl" beinhaltet gesättigte aliphatische (nicht-aromatische) Gruppen, einschließlich geradkettiger (linearer) Alkylgruppen (z.B. Methyl, Ethyl, Propyl, Butyl, Pentyl, Hexyl, Heptyl und Octyl) und verzweigter Alkylgruppen (z.B. Isopropyl, tert-Butyl, Isobutyl). Der Begriff umfasst auch O-, N-, S- oder P-Alkylgruppen (z.B. -O-Methyl), d.h. Alkylgruppen, die über ein Sauerstoff-, Stickstoff-, Schwefel- oder Phosphoratom an eine Verbindung gebunden sind.

Der Begriff "Alkenyl" beinhaltet ungesättigte aliphatische (nicht-aromatische) Gruppen mit mindestens einer C-C-Doppelbindung, einschließlich geradkettiger und verzweigter Alkenylgruppen. Der Begriff umfasst auch O-, N-, S- oder P-Alkenylgruppen (z.B. -O-Propenyl), d.h. Alkenylgruppen, die über ein Sauerstoff-, Stickstoff-, Schwefel- oder Phosphoratom an eine Verbindung gebunden sind.

Der Begriff "Alkinyl" beinhaltet ungesättigte aliphatische (nicht-aromatische) Gruppen mit mindestens einer C-C-Dreifachbindung, einschließlich geradkettiger und verzweigter Alkenylgruppen. Der Begriff umfasst auch O-, N-, S- oder P-Alkinylgruppen (z.B. -O-Butinyl), d.h. Alkinylgruppen, die über ein Sauerstoff-, Stickstoff-, Schwefel- oder Phosphoratom an eine Verbindung gebunden sind.

Der Begriff "Alkeninyl" beinhaltet ungesättigte aliphatische (nicht-aromatische) Gruppen mit mindestens einer C-C-Doppelbindung und mindestens einer C-C-Dreifachbindung, einschließlich geradkettiger und verzweigter Alkeninylgruppen. Der Begriff umfasst auch O-, N-, S- oder P-Alkeninylgruppen, d.h. Alkeninylgruppen, die über ein Sauerstoff-, Stickstoff-, Schwefel- oder Phosphoratom an eine Verbindung gebunden sind.

Der Begriff "Cycloalkyl" beinhaltet alicyclische Gruppen, d.h. ringförmige gesättigte aliphatische (nicht-aromatische) Gruppen, z.B. Cyclopropyl, Cyclopentyl, Cyclohexyl, Cycloheptyl, Cyclooctyl. Der Begriff umfasst auch O-, N-, S- oder P-Cycloalkylgruppen, d.h. Cycloalkylgruppen, die über ein Sauerstoff-, Stickstoff-, Schwefel- oder Phosphoratom an eine Verbindung gebunden sind. Die Begriffe "Cycloalkenyl", "Cycloalkinyl" und "Cycloalkeninyl" bedeuten entsprechend ringförmige aliphatische (nicht-aromatische) Alkenyle, Alkinyle oder Alkeninyle gemäß der obigen Definition, wobei die Doppel- und/oder Dreifachbindung(en) innerhalb oder außerhalb des Rings bzw. Ringsystems vorhanden sein kann (können).

Der Begriff "Heteroalkyl" bezeichnet Alkylgruppen, bei denen ein oder mehrere Kohlenstoffatome des Kohlenwasserstoffgerüsts durch andere Atome (Heteroatome), z.B. Sauerstoff-, Stickstoff-, Schwefel- oder Phosphoratome, ersetzt sind. Der Begriff umfasst auch O-, N-, S- oder P-Heteroalkylgruppen, d.h. Heteroalkylgruppen, die über ein Sauerstoff-, Stickstoff-, Schwefel- oder Phosphoratom an eine Verbindung gebunden sind. Der Begriff "Heteroalkyl" umfasst auch Cycloalkyle, bei denen ein oder mehrere Kohlenstoffatome des Kohlenwasserstoffgerüsts durch andere Atome, z.B. Sauerstoff-, Stickstoff-, Schwefel- oder Phosphoratome, ersetzt sind. Unter den Begriffen "Heteroalkenyl", "Heteroalkinyl", "Heteroalkeninyl" werden entsprechend Alkenyle, Alkinyle und Alkeninyle sowie Cycloalkenyle, Cycloalkinyle und Cycloalkeninyle verstanden, bei denen ein oder mehrere Kohlenstoffatome des Kohlenwasserstoffgerüsts durch andere Atome (Heteroatome), z.B. Sauerstoff-, Stickstoff-, Schwefel- oder Phosphoratome, ersetzt sind. Der Begriff "C₁₋₂₀-Heteroalkyl" bedeutet beispielsweise eine Alkylgruppe mit 1, 2, 3, 4, 5, 6, 7, 8, 9, 10, 11, 12, 13, 14, 15, 16, 17, 18, 19 oder 20 C-Atomen und mindestens einem Heteroatom. Entsprechendes gilt auch für Heteroalkenyle, Heteroalkinyle und Heteroalkeninyle.

Unter "Aryl" werden Gruppen mit Aromatizität verstanden, einschließlich mehrgliedrigen aromatischen Einzelringgruppen sowie multizyklischen Systemen mit mindestens einem aromatischen Ring. Beispiele für Aryl-Gruppen beinhalten Benzol, Phenyl und Naphthalen. Der Begriff umfasst auch O-, N-, S- oder P-Arylgruppen, d.h. Arylgruppen, die über ein Sauerstoff-, Stickstoff-, Schwefel- oder Phosphoratom an eine Verbindung gebunden sind. Die Begriffe "aromatischer Rest" oder "Aromatenrest" werden hier synonym zu "Aryl" verwendet.

Unter "Heteroaryl" werden Aryl-Gruppen verstanden, die Heteroatome in der Ringstruktur aufweisen, bei denen also ein oder mehrere Kohlenstoffatome in der Ringstruktur durch andere Atome (Heteroatome) ersetzt sind, z.B. durch Sauerstoff-, Stickstoff-, Schwefel- oder Phosphoratome. Beispiele für Heteroaryle sind Pyrrol, Furan, Thiophen, Thiazol, Isothiazol, Imidazol, Triazol, Tetrazol, Pyrazol, Oxazol, Pyridin, Pyrazin, Pyridazin und Pyrimidin. Der Begriff beinhaltet auch multizyklische Aryl-Gruppen, z.B. bizyklische und trizyklische, z.B. Benzoxazol, Benzodioxazol, Benzothiazol, Benzoimidazol, Benzothiophen, Methylendioxyphenyl, Quinolin, Isoquinolin, Indol, Benzofuran, Purin oder Benzofuran. Der Begriff umfasst auch O-, N-, S- oder P-Heteroarylgruppen, d.h. Heteroarylgruppen, die über ein Sauerstoff-, Stickstoff-, Schwefel- oder Phosphoratom an eine Verbindung gebunden sind. Die Begriffe "heteroaromatischer Rest" oder "Heteroaromatenrest" werden hier synonym zu "Heteroaryl" verwendet.

Unter dem Begriff "Halogen" wird hier Chlor (Cl), Fluor (F), Brom (Br) und Iod (I) verstanden, insbesondere Chlor (Cl) und Fluor (F) verstanden.

Der Begriff "substituiert" bedeutet, dass ein oder mehrere Substituenten vorhanden sind, die ein Wasserstoffatom an einem oder mehreren Kohlenstoffatomen des Kohlenwasserstoffgerüsts oder an einem oder mehreren Heteroatomen im Kohlenstoffgerüst ersetzen. Beispiele für solche Substituenten sind Oxo-, Hydroxyl-, Phosphat-, Cyano-, Azido- und Aminogruppen, aber auch z.B. Halogene (z.B. F, Cl), Alkyl-, Cycloalkyl-, Heteroalkyl-, Heterocycloalkyl-, Aryl- und Heteroarylgruppen.

Unter einem "Elektronenakzeptor" wird hier eine Verbindung, ein Verbindungsrest oder funktionelle Gruppe verstanden, die Elektronen zu sich heranzieht und damit in einer Verbindung eine Ladungsverschiebung, d.h. Polarisierung, herbeiführt. Elektronenakzeptoren bzw. Elektronenakzeptorgruppen sind dem Fachmann bekannt und weisen beispielsweise einen negativen induktiven oder mesomeren Effekt auf. Beispiele für Elektronenakzeptorgruppen sind OMe, MeSO₂, =O, C(O)H, COOH, CN, SO₃H, Ketone, Ester bzw. die Estergruppe, NO₂ und Halogen (z.B. F, Cl). Me steht für Methyl.

Der Rest R⁹ ist in jedem Fall über ein Sauerstoffatom gebunden, im Falle eines Nucleosidmonophosphats oder Nucleosidmonophosphatanalogons vorzugsweise über ein Sauerstoff der Phosphat- bzw. Phosphonatgruppe. Zur Vermeidung von Missverständnissen sei klargestellt, dass OP(O)(OH)-R¹⁰ und OP(O)(OH)O-R¹⁰ Reste gemäß den folgenden Formeln 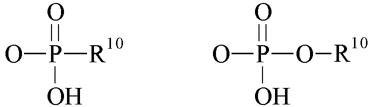 bedeuten, wobei R¹⁰ wie oben definiert ist. Weiter zur Vermeidung von Missverständnissen sei klargestellt, dass das Nukleosid oder Nukleosidanalogon über ein Sauerstoffatom der Zuckerkomponente, beispielsweise am C5-Atom einer Pentose, an das jeweilige Phosphoratom gebunden ist. Im Falle einer Pentose erfolgt die Verknüpfung bevorzugt über die OH-Gruppe am 5'-C-Atom. Die Verknüpfung kann aber auch über ein Sauerstoffatom am 2'- oder 3'-C-Atom erfolgen. O-R¹⁰ wird hier gegebenenfalls auch als "Alkoholrest" bezeichnet, wobei hierunter jede organische Kohlenstoffverbindung verstanden wird, bei der ein Wasserstoffatom am Kohlenstoffgerüst durch eine Hydroxylgruppe ersetzt ist. Beispiele für Alkohole umfassen Zucker wie Glukose, Fruktose, Mannose etc, aber auch andere Verbindungen wie z.B. Geraniol.

In einer bevorzugten Ausführungsform der Erfindung sind die Reste R¹, R³, R⁵ und R⁷ bei der Verbindung gemäß der obigen Formel I jeweils unabhängig voneinander H, Halogen, NO₂, CN, SO₃H, oder ein cyclischer, acyclischer, linearer oder verzweigter C₁₋₂₀-Aliphatrest oder C₁₋₂₀-Heteroaliphatrest, oder ein C₅₋₂₀-Aromatenrest oder C₃₋₂₀-Heteroaromatenrest, besonders bevorzugt unabhängig voneinander H, oder ein cyclischer, acyclischer, linearer oder verzweigter C₁₋₁₀-Aliphatrest oder C₁₋₁₀-Heteroaliphatrest, oder ein C₅₋₁₂-Aromatenrest oder C₃₋₁₂-Heteroaromatenrest.

In einer weiteren bevorzugten Ausführungsform der Erfindung sind R¹, R³, R⁵ und R⁷ bei der Verbindung gemäß der obigen Formel I jeweils unabhängig voneinander ausgewählt aus der Gruppe bestehend aus H, Halogen, NO₂, CN, SO₃H, C₁₋₂₀-Alkyl, C₂₋₂₀-Alkenyl, C₂₋₂₀-Alkinyl, C₄₋₂₀-Alkeninyl, C₃₋₂₀-Cycloalkyl, C₃₋₂₀-Cycloalkenyl, C₅₋₂₀-Cycloalkinyl, C₅₋₂₀-Cycloalkeninyl, C₁₋₂₀-Heteroalkyl, C₂₋₂₀-Heteroalkenyl, C₂₋₂₀-Heteroalkinyl, C₄₋₂₀-Heteroalkeninyl, C₅₋₂₄-Aryl, C₃₋₂₄-Heteroaryl.

Weiter bevorzugt sind R¹, R³, R⁵ und R⁷ bei der Verbindung gemäß der obigen Formel I jeweils unabhängig voneinander ausgewählt aus der Gruppe bestehend aus H, Halogen, NO₂, CN, SO₃H, C₁₋₁₀-Alkyl, C₂₋₁₀-Alkenyl, C₂₋₁₀-Alkinyl, C₄₋₁₀-Alkeninyl, C₃₋₁₀-Cycloalkyl, C₃₋₁₀-Cycloalkenyl, C₅₋₁₀-Cycloalkinyl, C₅₋₁₀-Cycloalkeninyl, C₁₋₁₀-Heteroalkyl, C₂₋₁₀-Heteroalkenyl, C₂₋₁₀-Heteroalkinyl, C₄₋₁₀-Heteroalkeninyl, C₅₋₁₂-Aryl, C₃₋₁₂-Heteroaryl. Besonders bevorzugt sind R¹, R³, R⁵ und R⁷ sämtlich H.

R¹⁰ ist vorzugsweise ein cyclischer, acyclischer, linearer oder verzweigter C₁₋₂₀-Aliphatrest oder C₁₋₂₀-Heteroaliphatrest, oder ein C₅₋₂₀-Aromatenrest oder C₃₋₂₀-Heteroaromatenrest, besonders bevorzugt unabhängig voneinander H, oder ein cyclischer, acyclischer, linearer oder verzweigter C₁₋₁₀-Aliphatrest oder C₁₋₁₀-Heteroaliphatrest, oder ein C₅₋₁₂-Aromatenrest oder C₃₋₁₂-Heteroaromatenrest, weiter bevorzugt C₁₋₂₀-Alkyl oder C₁₋₂₀-Alkenyl oder ein Zuckerrest.

Die Reste R¹², R¹³, R¹⁴ und R¹⁵ sind vorzugsweise
i. jeweils unabhängig voneinander H, ein cyclischer, acyclischer, linearer oder verzweigter C₁₋₂₀-Aliphatrest oder C₁₋₂₀-Heteroaliphatrest, ein C₅₋₂₀-Aromatenrest oder C₃₋₂₀-Heteroaromatenrest, und/oder ein Elektronenakzeptor, oder
ii. jeweils unabhängig voneinander H, ein cyclischer, acyclischer, linearer oder verzweigter C₁₋₁₀-Aliphatrest oder C₁₋₁₀-Heteroaliphatrest, oder ein C₅₋₁₂-Aromatenrest oder C₃₋₁₂-Heteroaromatenrest, oder
iii. jeweils unabhängig voneinander ausgewählt aus der Gruppe bestehend aus H, C₁₋₂₀-Alkyl, C₂₋₂₀-Alkenyl, C₂₋₂₀-Alkinyl, C₄₋₂₀-Alkeninyl, C₃₋₂₀-Cycloalkyl, C₃₋₂₀-Cycloalkenyl, C₅₋₂₀-Cycloalkinyl, C₅₋₂₀-Cycloalkeninyl, C₁₋₂₀-Heteroalkyl, C₂₋₂₀-Heteroalkenyl, C₂₋₂₀-Heteroalkinyl, C₄₋₂₀-Heteroalkeninyl, C₅₋₂₄-Aryl, C₃₋₂₄-Heteroaryl, oder
iv. jeweils unabhängig voneinander ausgewählt aus der Gruppe bestehend aus H, C₁₋₁₀-Alkyl, C₂₋₁₀-Alkenyl, C₂₋₁₀-Alkinyl, C₄₋₁₀-Alkeninyl, C₃₋₁₀-Cycloalkyl, C₃₋₁₀-Cycloalkenyl, C₅₋₁₀-Cycloalkinyl, C₅₋₁₀-Cycloalkeninyl, C₁₋₁₀-Heteroalkyl, C₂₋₁₀-Heteroalkenyl, C₂₋₁₀-Heteroalkinyl, C₅₋₁₂-Aryl, C₃₋₁₂-Heteroaryl, oder
v. alle H, oder
vi. ein Elektronenakzeptor oder H, mit der Maßgabe, dass R¹² und R¹⁴ jeweils H und R¹³ und R¹⁵ jeweils ein Elektronenakzeptor sind, oder R¹³ und R¹⁵ jeweils H und R¹² und R¹⁴ jeweils ein Elektronenakzeptor sind.

Bevorzugt unterscheiden sich die beiden Masken "A" und "B" bei der vorliegenden Erfindung nur in den Resten R² und/oder R⁴ sowie R⁶ und/oder R⁸. Die übrigen Reste, d.h. die Reste R¹, R³, R⁵ und R⁷ sowie vorzugsweise auch die Reste R¹², R¹³, R¹⁴ und R¹⁵ sind vorzugsweise alle gleich, besonderes bevorzugt alle H. Besonders bevorzugt unterscheiden sich die beiden Masken nur in ihren Resten R² und R⁶ oder nur in ihren Resten R⁴ und R⁸, bevorzugt nur in ihren Resten R² und R⁶.

R² und R⁴ sind unabhängig voneinander H oder Z-C(Y)-R_{A}, wobei die Bindung an den Phenylring über Z erfolgt, und wobei Z und Y für O, S oder HN stehen, mit der Maßgabe, dass nicht beide Reste R² und R⁴ jeweils H sind. Gleiches gilt für die Reste R⁶ und R⁸. Für den Fall, dass Y = O ist, kommen für die Reste R², R⁴, R⁶ und R⁸ beispielsweise folgende Strukturen in Frage:

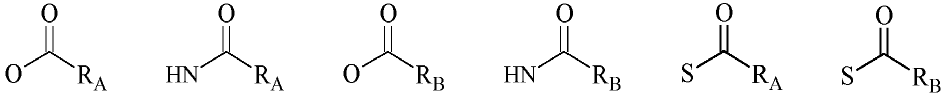

Vorzugsweise ist nur einer der Reste R² und R⁴, bevorzugt R², Z-C(Y)-R_{A}, und der jeweils andere Rest, bevorzugt R⁴, ist H, wobei Z-C(Y)-R_{A} vorzugsweise Z-C(O)-R_{A} ist. Gleichermaßen ist vorzugsweise nur einer der Reste R⁶ und R⁸, bevorzugt R⁶, Z-C(Y)-R_{B}, und der jeweils andere Rest, bevorzugt R⁸, ist H, wobei Z-C(Y)-R_{B} vorzugsweise Z-C(O)-R_{B} ist.

Bei einer besonders bevorzugten Ausführungsform der erfindungsgemäßen Verbindung I ist Rest R² = Z-C(O)-R_{A}, und R⁶ ist Z-C(O)-R_{B}, während die Reste R¹, R³, R⁴, R⁵, R⁷, R⁸, R¹², R¹³, R¹⁴ und R¹⁵ jeweils H sind, wie in der folgenden Verbindung gemäß der Formel Ic: 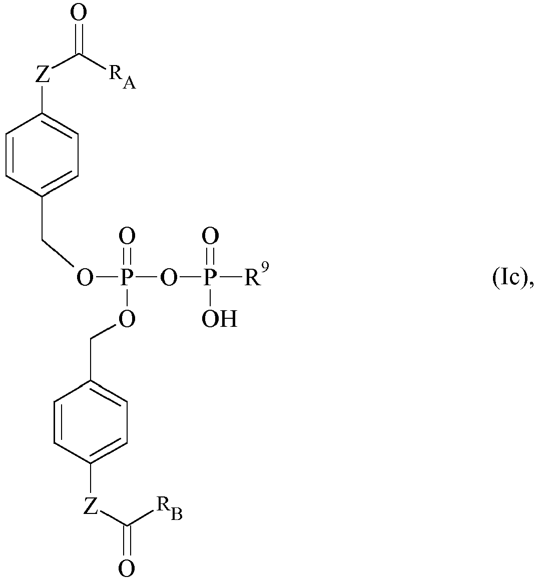 wobei Z = O, S oder HN, vorzugsweise O, und R⁹ wie oben definiert ist.

Bei der erfindungsgemäßen Verbindung gemäß der allgemeinen Formel I sind die Reste R_{A} und R_{B} vorzugsweise jeweils unabhängig voneinander ein cyclischer, acyclischer, linearer oder verzweigter C₁₋₂₀-Aliphatrest oder C₁₋₂₀-Heteroaliphatrest, oder ein C₅₋₂₀-Aromatenrest oder C₃₋₂₀-Heteroaromatenrest, mit der Maßgabe, dass die Reste R_{A} und R_{B} verschieden sind. Besonders bevorzugt sind die Reste R_{A} und R_{B} jeweils unabhängig voneinander ausgewählt aus der Gruppe bestehend aus C₁₋₂₀-Alkyl, C₂₋₂₀-Alkenyl, C₂₋₂₀-Alkinyl, C₄₋₂₀-Alkeninyl, C₃₋₂₀-Cycloalkyl, C₃₋₂₀-Cycloalkenyl, C₅₋₂₀-Cycloalkinyl, C₅₋₂₀-Cycloalkeninyl, C₁₋₂₀-Heteroalkyl, C₂₋₂₀-Heteroalkenyl, C₂₋₂₀-Heteroalkinyl, C₄₋₂₀-Heteroalkeninyl, C₅₋₂₄-Aryl, C₃₋₂₄-Heteroaryl, vorzugsweise C₁₋₂₀-Alkyl oder C₁₋₂₀-Alkenyl, mit der Maßgabe, dass die Reste R_{A} und R_{B} verschieden sind. Besonders bevorzugt sind die Reste R_{A} und R_{B} ausgewählt aus C₁₋₂₀-Alkyl und C₂₋₂₀-Alkenyl, mit der Maßgabe, dass die Reste R_{A} und R_{B} verschieden sind.

Vorzugsweise ist der Rest R_{B} dabei so ausgewählt, dass er im Vergleich zu R_{A} lipophiler ist. Umgekehrt wird der Rest R_{A} dabei vorzugsweise so ausgewählt, dass er instabiler ist, d.h. eher enzymatische abgespalten wird. Beispielsweise kann R_{A} ein C₁₋₅-Alkyl sein, während R_{B} ein C₇₋₂₀-Alkyl ist. Beispielsweise kann R_{A} Methyl (Me), Propyl (Pr) oder Butyl (Bu) sein, während R_{B} Ph-CF₃, Ph-Me, C₇H₁₅, C₉H₁₉, C₁₁H₂₃ oder 5-OAc-Man ist (Ph = Phenyl, Me = Methyl, OAc = Acetoxygruppe, Man = Mannose).

Die Erfindung betrifft daher in einem weiteren Aspekt auch eine pharmazeutische Zusammensetzung, die eine erfindungsgemäße Verbindung und einen pharmazeutisch annehmbaren Träger umfasst. Pharmazeutisch annehmbare Träger sind dem Fachmann bekannt und umfassen einen oder mehrere flüssige, halbfeste oder feste Füllstoffe, Verdünnungsmittel oder andere Substanzen, welche für eine Verabreichung an Säuger, einschließlich des Menschen, geeignet sind.

Der Ausdruck "Träger" im Sinne der vorliegenden Erfindung bezeichnet dabei jeglichen organischen oder anorganischen, natürlichen oder synthetischen Stoff, welcher zur Vereinfachung der Applikation mit dem Wirkstoff kombiniert werden kann. Beispiele für derartige Träger umfassen, sind jedoch nicht beschränkt auf, organische oder anorganische Lösungsmittel, Stärke, Laktose, Mannitol, Methylcellulose, Talk, Gelatine, Agar-Agar, Calciumphosphat, Magnesiumstearat, tierische und pflanzliche Fette, höhermolekulare Fettsäuren, oder höhermolekulare Polymere.

Der Ausdruck "pharmazeutisch annehmbar" bezeichnet jegliches für Säuger, insbesondere den Menschen, im Wesentlichen nichttoxische Material, welches die Wirksamkeit der biologischen Aktivität des Wirkstoffes im Wesentlichen nicht beeinträchtigt. Derartige Materialien können pharmazeutisch annehmbare Konzentrationen an Salzen, Puffern, Konservierungsstoffen, oder dergleichen umfassen. Nicht-limitierende Beispiele pharmazeutisch annehmbarer Träger schließen Magnesiumcarbonat, Magnesiumstearat, Talk, Zucker, Laktose, Ethanol, Glycerin, Wasser, Pufferlösungen usw. ein. Die pharmazeutische Zusammensetzung kann darüber hinaus auch noch Hilfs- und/oder Verdünnungsmittel umfassen.

In einem weiteren Aspekt betrifft die Erfindung auch eine pharmazeutische Darreichungsform, die eine erfindungsgemäße Verbindung und einen pharmazeutisch annehmbaren Träger umfasst. Besonders bevorzugt handelt es sich dabei um eine Darreichungsform zur oralen Verabreichung, beispielsweise eine Tablette oder Kapsel.

In noch einem weiteren Aspekt betrifft die vorliegende Erfindung ein Verfahren zur Herstellung einer Verbindung der allgemeinen Formel (I) 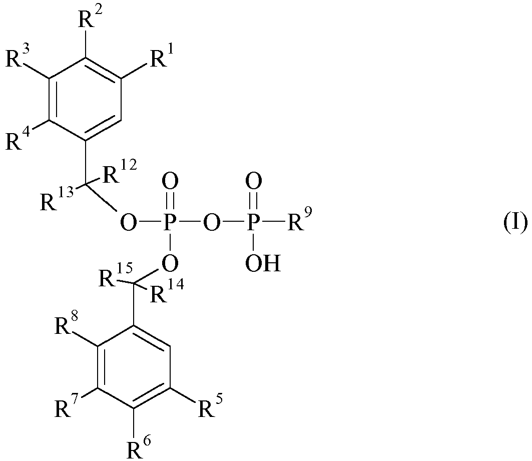 oder eines pharmazeutisch annehmbaren Salzes davon, wobei
R¹, R³, R⁵ und R⁷ unabhängig voneinander H, Halogen, NO₂, CN, SO₃H, oder ein substituierter oder unsubstituierter cyclischer, acyclischer, linearer oder verzweigter aliphatischer oder heteroaliphatischer Rest, oder ein substituierter oder unsubstituierter aromatischer oder heteroaromatischer Rest sind,
R² und R⁴ unabhängig voneinander H oder Z-C(Y)-R_{A}, jedoch nicht beide H sind,
R⁶ und R⁸ unabhängig voneinander H, Z-C(Y)-R_{B}, jedoch nicht beide H sind,
Z, Y unabhängig voneinander O, S oder HN sind,
R_{A} und R_{B} verschieden und jeweils ein substituierter oder unsubstituierter cyclischer, acyclischer, linearer oder verzweigter aliphatischer oder heteroaliphatischer Rest, oder ein substituierter oder unsubstituierter aromatischer oder heteroaromatischer Rest sind,
R⁹ Nukleosid, Nukleosidmonophosphat, Nukleosidanalogon, Nukleosidmonophosphatanalogon, O-R¹⁰, OP(O)(OH)-R¹⁰ oder OP(O)(OH)O-R¹⁰ ist, wobei R¹⁰ ein substituierter oder unsubstituierter cyclischer, acyclischer, linearer oder verzweigter aliphatischer oder heteroaliphatischer Rest, oder ein substituierter oder unsubstituierter aromatischer oder heteroaromatischer Rest ist, und wobei
R¹² , R¹³, R¹⁴ und R¹⁵ unabhängig voneinander H, ein substituierter oder unsubstituierter cyclischer, acyclischer, linearer oder verzweigter aliphatischer oder heteroaliphatischer Rest, ein substituierter oder unsubstituierter aromatischer oder heteroaromatischer Rest, und/oder ein Elektronenakzeptor sind,
umfassend die folgenden Schritte:
a₁) Inreaktionbringen einer Verbindung der allgemeinen Formel II_{A} 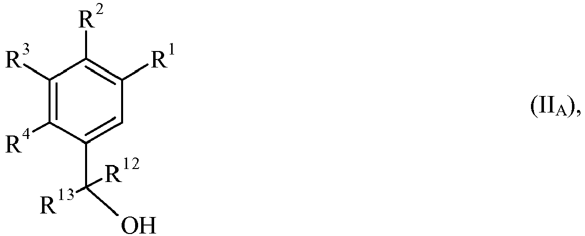 wobei R¹, R², R³, R⁴, R¹² und R¹³ wie oben definiert sind,
   mit i) Phosphortrichlorid PCl₃ und *N*,*N*-Di*iso*propylamin, oder ii) Bis(N,N-diisopropylamino)chlorphosphin
   zur Synthese einer Verbindung der allgemeinen Formel III 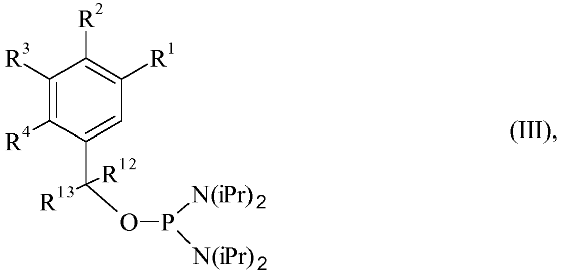
b₁) Inreaktionbringen der in a) erhaltenen Verbindung der Formel III mit einer Verbindung der allgemeinen Formel II_{B} 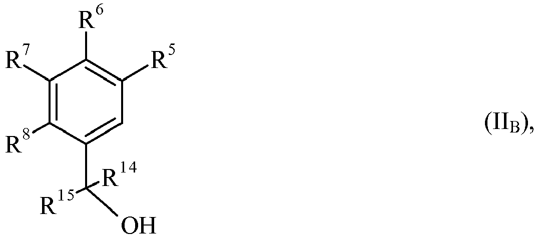 wobei R⁵, R⁶, R⁷, R⁸, R¹⁴ und R¹⁵ wie oben definiert sind,
   zur Synthese einer Verbindung der allgemeinen Formel IV 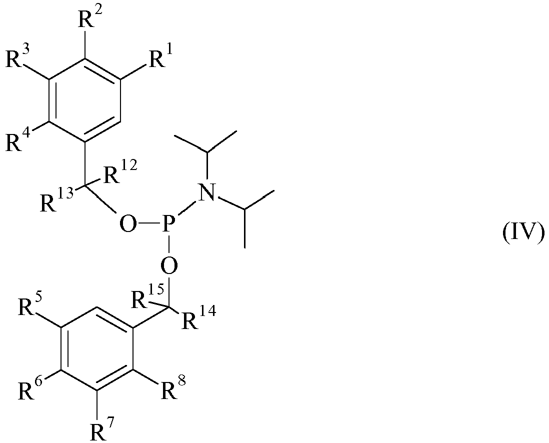
c₁) Inreaktionbringen der in b) erhaltenen Verbindung der Formel IV mit einer Verbindung gemäß der allgemeinen Formel V 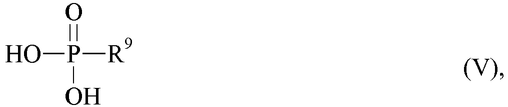 wobei R⁹ wie oben definiert ist.

In einer alternativen Ausgestaltung umfasst das erfindungsgemäße Verfahren die folgenden Schritte:
a₂) Inreaktionbringen einer Verbindung der allgemeinen Formel II_{A} 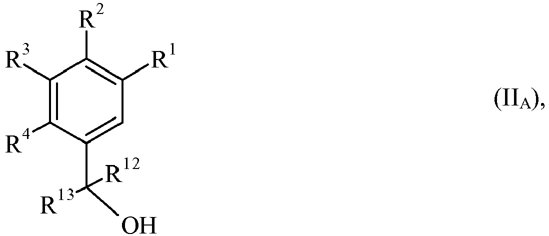 wobei R¹, R², R³, R⁴, R¹², und R¹³ wie oben definiert sind,
   mit Diphenylphosphonat zur Synthese einer Verbindung der allgemeinen Formel VI 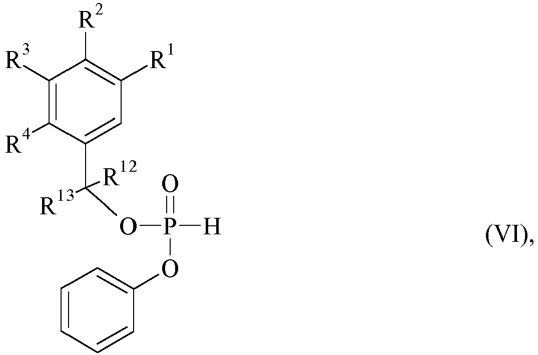
b₂) Inreaktionbringen der in a) erhaltenen Verbindung der Formel VI mit einer Verbindung der allgemeinen Formel II_{B} 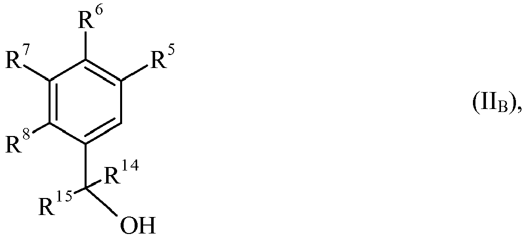 wobei R⁵, R⁶, R⁷, R⁸, R¹⁴ und R¹⁵ wie oben definiert sind,
   zur Synthese einer Verbindung der allgemeinen Formel VII 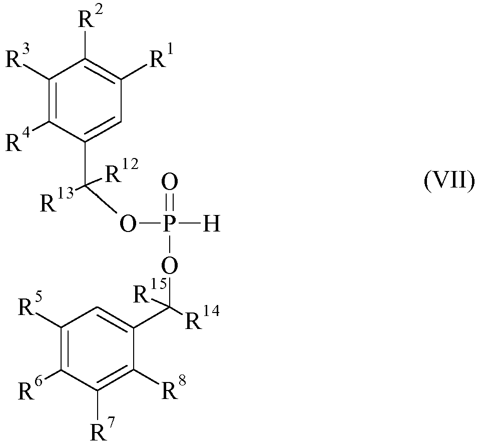
c₂) Inreaktionbringen der in b) erhaltenen Verbindung der Formel VII mit i) einer Verbindung gemäß der allgemeinen Formel V 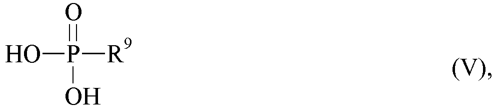 oder ii) mit Phosphorsäure oder einem Phosphorsäuresalz und R⁹ oder einer R⁹ umfassenden Verbindung,
   wobei R⁹ wie oben definiert ist.

Ein Beispiel für ein geeignetes Phosphorsäuresalz ist Tetrabutylammoniumphosphat.

Die alternative Ausgestaltung des erfindungsgemäßen Verfahrens ist besonders vorteilhaft, weil es dadurch möglich ist, im Fall der Synthese von Nukleosiddi- und -triphosphaten oder deren Analoga auf den Einsatz der häufig schwer herstellbaren Diphosphate bzw. deren Analoga zu verzichten und statt dessen entsprechende Monophosphate zu verwenden, die leichter herstellbar und teils auch kommerziell verfügbar sind. Bei dieser Variante wird die Verbindung gemäß Formel VII entweder direkt mit einer Monophosphatverbindung zur Diphosphatverbindung umgesetzt, oder zunächst zur entsprechenden Pyrophosphatverbindung phosphoryliert, welche anschließend mit einer Monophosphatverbindung umgesetzt werden kann. Auf diese Weise sind Di- und insbesondere Triphosphatverbindungen erhältlich, ohne dass Diphosphatverbindungen eingesetzt werden müssen. Selbstverständlich ist aber auch bei dieser Variante, auch wenn dies nicht bevorzugt ist, die Verwendung von Diphosphatverbindungen zur Herstellung entsprechender Triphosphatverbindungen nicht ausgeschlossen.

Bevorzugte Ausgestaltungen der Reste R¹ bis R¹⁵ wurden bereits oben für den ersten Aspekt der vorliegenden Erfindung angegeben und gelten ohne Abstriche auch für die Verfahrensaspekte der Erfindung, sodass hier auf eine Wiederholung verzichtet wird.

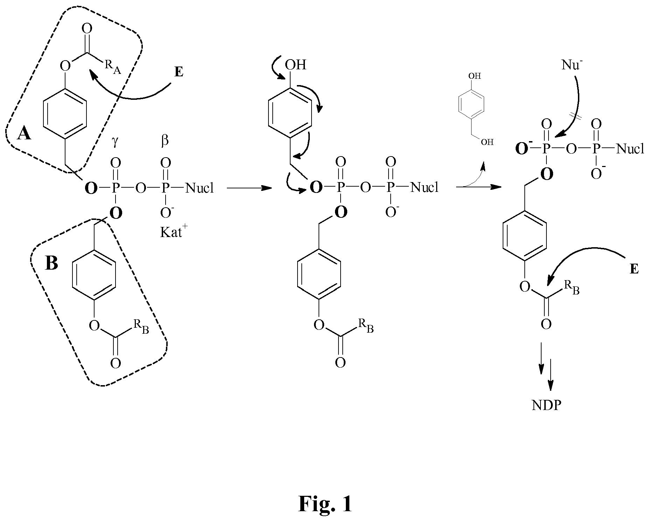

Die Erfindung wird im Folgenden anhand der Figur 1 sowie Ausführungsbeispielen allein zu Veranschaulichungszwecken näher erläutert. Es zeigt
Figur 1 Schematische Darstellung der intrazellulären Abspaltung der Masken bei einer Ausführungsform der erfindungsgemäßen Verbindung.

Figur 1 zeigt schematisch den angenommen Verlauf der Freisetzung eines Nukleosiddiphophat-Propharmakons (NDP-Propharmakon oder NDP-Prodrug) gemäß einer Ausführungsform der vorliegenden Erfindung in der Zelle. Die beiden ansonsten negativ geladenen Sauerstoffatome (in Fettdruck) der Hydroxylgruppen des endständigen Phosphats (hier das β-Phosphoratom) in dem NDP-Propharmakon sind asymmetrisch substituiert, d.h. mit zwei verschiedenen Masken A und B (hier unterschiedlichen Acyloxybenzyl-Derivaten) maskiert. Hierzu unterscheiden sich die Reste R_{A} und R_{B} in ihrer Stabilität und Lipophilität. Der Rest R_{A} weist eine geringere Stabilität auf als der Rest R_{B}, der Rest R_{B} ist lipophiler als der Rest R_{A}. In der Zelle wird die vergleichsweise instabile Maske A nach einem schnell erfolgenden enzymatischen Angriff auf die Estergruppe durch in der Zelle vorhandene Esterasen (E) und anschließenden spontanen Abbau rasch entfernt, sodass ein monomaskiertes Intermediat resultiert. Ein nucleophiler Angriff (Nu⁻ = Nucleophil) auf die Phosphoranhydridbindung mit einer Spaltung derselben erfolgt nicht, sodass die unerwünschte Bildung des Monophosphats verhindert wird. Die Maske B wird durch die gleichen Mechanismen wie die Maske A, jedoch in einem langsamer verlaufenden Prozess, ebenfalls abgespalten, sodass das unmaskierte Nukleosiddiphosphat-Propharmakon schließlich in der Zelle freigesetzt wird.

### Ausführungsbeispiele

Ein allgemeines Schema für eine Ausführungsform eines erfindungsgemäßen Verfahrens zur Herstellung erfindungsgemäßer Verbindungen I ist im Folgenden wiedergegeben. Ein erstes Benzylalkoholderivat **II_{A}** wird mit Phosphortrichlorid (PCl₃) in Reaktion gebracht, um die Verbindung **100** herzustellen, die mit *N*,*N*-Di*iso*propylamin (NH(*i*Pr)₂) zur entsprechenden Phosphordiamidit-Verbindung **III** umgesetzt wird. Die Verbindung III wird dann mit einem zweiten Benzylalkoholderivat **II_{B}** in Reaktion gebracht, um die Phosphoramiditverbindung **IV** zu synthetisieren. Diese kann dann mit einer Mono- oder Diphosphatverbindung **V**, beispielsweise einem Nukleosidmonophosphat bzw. Nukleosidmonophosphatanalogon, die hier gegebenenfalls beide mit NMP abgekürzt sind, oder Nukleosiddiphosphat bzw. Nukleosiddiphosphatanalogon, die hier gegebenenfalls beide als NDP bezeichnet sind, gekoppelt werden, wodurch die Verbindung **I** erhalten wird. Erfindungsgemäße NDP- oder NTP-Verbindungen (einschließlich Nukleosidanaloga enthaltender Verbindungen) werden hier gegebenenfalls auch kurz als DiPPro-Nucleotide oder TriPPPro-Nucleotide bezeichnet.

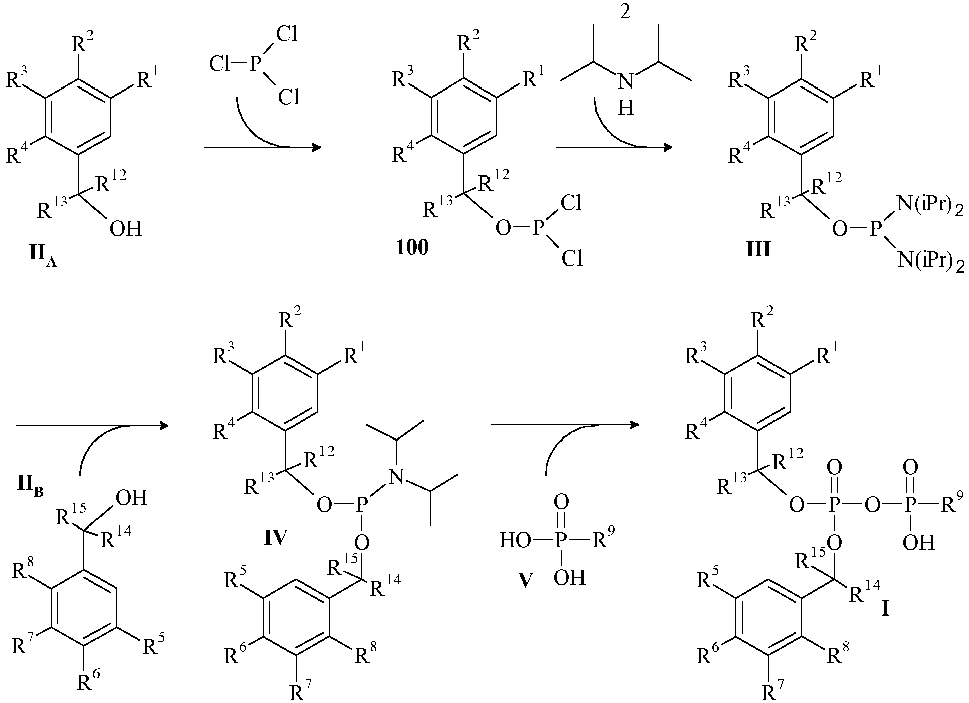

Zur Herstellung der Phosphordiamiditverbindung **III** kann das Benzylalkoholderivat **II_{A}** statt mit Phosphortrichlorid (PCl₃) und *N*,*N*-Di*iso*propylamin alternativ auch mit Bis(*N*,*N-*di*iso*propylamino)chlorphosphin gemäß dem folgenden allgemeinen Schema in Reaktion gebracht werden.

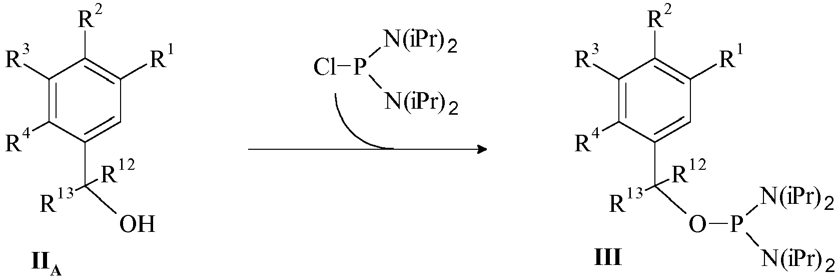

Ein allgemeines Schema zu einer alternativen Ausführungsform eines erfindungsgemäßen Verfahrens zur Herstellung erfindungsgemäßer Verbindungen **I** ist im Folgenden wiedergegeben.

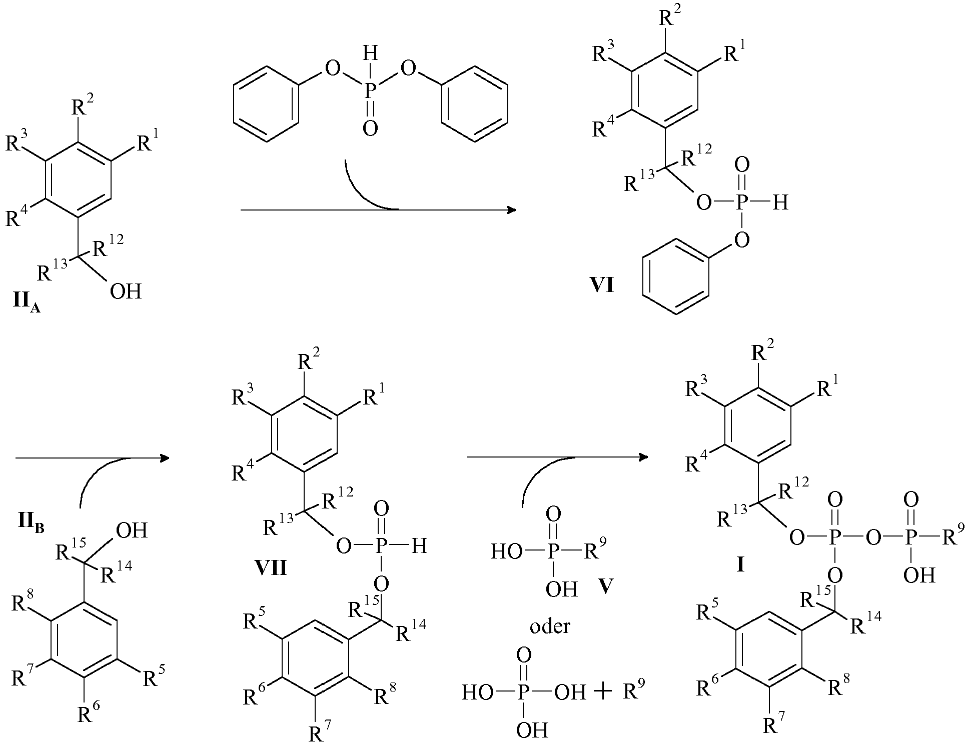

Ein erstes Benzylalkoholderivat **II_{A}** wird mit Diphenylphosphonat (DPP) in Reaktion gebracht, um die Verbindung **VI** herzustellen, die mit einem zweiten Benzylalkoholderivat **II_{B}** in Reaktion gebracht wird, um die Phosphonatverbindung **VII** zu synthetisieren. Diese kann dann mit einer Mono- oder Diphosphatverbindung **V**, oder zunächst mit Phosphorsäure oder einem Phosphorsäuresalz und anschließend mit einer Verbindung R⁹ oder einer R⁹ umfassenden Verbindung, beispielsweise mit einem Nukleosidmonophosphat, gekoppelt werden, wodurch die Verbindung **I** erhalten wird. Bei der letzteren Variante dieser Ausführungsform des erfindungsgemäßen Verfahrens ist besonders vorteilhaft, dass die Synthese von Triphosphatverbindungen mit den entsprechenden Monophosphaten, z.B. Nukleosidmonophosphaten oder Analoga davon, erfolgen kann.

Beispiele für mit den erfindungsgemäßen Verfahren hergestellte Di*PP*ro-Nucleotide sind im Folgenden wiedergegeben:
a) Stavudin (d4T) als Nukleosidanalogonrest (Ph = Phenyl; Me = Methyl, OAc = Acetoxy):

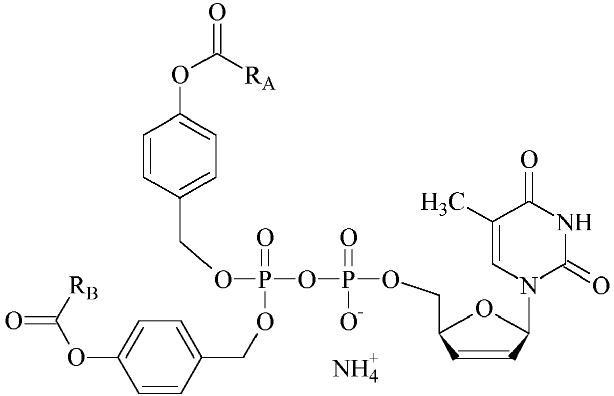

| **R_{A}** | **R_{B}** |
|---|---|
| CH₃ | PhCF₃ |
| CH₃ | Ph-Me |
| CH₃ | C₇H₁₅ |
| CH₃ | C₉H₁₉ |
| CH₃ | C₁₁H₂₃ |
| (CH₂)₃CH₃ | PhCF₃ |
| (CH₂)₃CH₃ | Ph-Me |
| (CH₂)₃CH₃ | C₇H₁₅ |
| (CH₂)₃CH₃ | C₉H₁₉ |
| (CH₂)₃CH₃ | C₁₁H₂₃ |

b) Zidovudin (AZT) als Nukleosidanalogonrest:

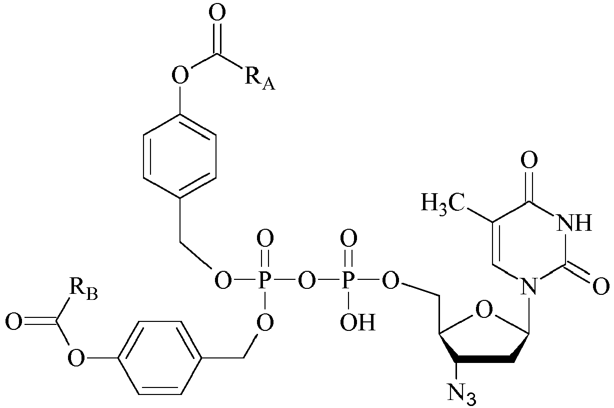

| **R_{A}** | **R_{B}** |
|---|---|
| CH₃ | PhCF₃ |
| CH₃ | C₇H₁₅ |
| CH₃ | C₉H₁₉ |
| CH₃ | C₁₁H₂₃ |
| (CH₂)₃CH₃ | PhCF₃ |
| (CH₂)₃CH₃ | C₇H₁₅ |
| (CH₂)₃CH₃ | C₉H₁₉ |
| (CH₂)₃CH₃ | C₁₁H₂₃ |

### 1. Herstellung asymmetrisch maskierter Nukleosiddi- und Nukleosidtriphosphat-Verbindungen über Phosphordiamiditverbindungen

Zur Herstellung erfindungsgemäßer asymmetrisch maskierter Nukleosiddi- oder Nukleosidtriphosphat-Verbindungen (Di*PP*ro-, Tri*PPP*ro-Nukleotide) können entsprechend den oben beschriebenen allgemeinen Verfahren zunächst die jeweiligen asymmetrischen Phosphoramidite hergestellt werden, um anschließend eine Kupplung mit dem gewünschten Nucleotid(analogon) (Nukleosidmono- oder Nukleosiddiphosphat(analogon)) zum asymmetrischen NDP oder NTP durchzuführen. Auf diesem Wege konnte eine Vielzahl asymmetrisch maskierter Nukleosiddi- oder Nukleosidtriphosphat-Verbindungen in sehr guten Ausbeuten darstellt werden. Die Kupplung der asymmetrischen Phosphoramidite mit den jeweiligen Nukleosidmono- bzw. -diphosphaten wurde in einer sauer-aktivierten Reaktion optimiert, sodass viele Kombinationen von Nukleosiddi- oder Nukleosidtriphosphat-Verbindungen in quantitativen Umsätzen und in hohen isolierten Ausbeuten zugänglich sind.

### 1.1 Synthese von Phosphordiamiditen

### 1.1.1 Syntheseweg 1

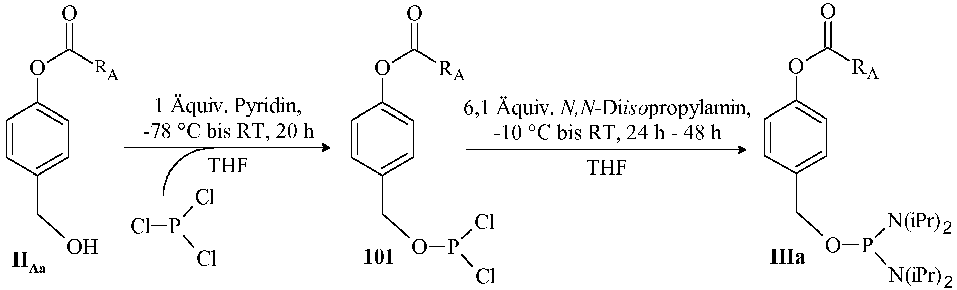

Unter Stickstoffatmosphäre werden 1 Äquivalent Phosphortrichlorid und 1 Äquivalent Pyridin in reichlich Tetrahydrofuran (THF, ca. 20 mL/g PCl₃) gelöst und auf-78 °C gekühlt. Zu dieser Lösung wird tropfenweise über einen Zeitraum von 1,5 h 1 Äquivalent des Phenylesters **II_{Aa}**, ebenfalls gelöst in THF, hinzugegeben. Nach dem Zutropfen wird das Kühlbad entfernt und das Reaktionsgemisch ca. 20 h bei Raumtemperatur (RT) gerührt, bis kein Phenylester mehr durch DC nachweisbar ist. Dann erfolgt die tropfenweise Zugabe von 6,1 Äquivalenten N,N-Diisopropylamin bei -10 °C. Nach Entfernen des Kühlbades wird das Reaktionsgemisch bei Raumtemperatur gerührt. Nach vollständiger Umsetzung der intermediären Verbindung **101**, d.h. nach ca. 24 bis 48 h, werden die gebildeten Salze durch Filtration und anschließend das Lösungsmittel unter vermindertem Druck entfernt. Ohne weitere Aufarbeitung erfolgt die Reinigung des Produkts **IIIa** am Chromatotron® mit Petrolether/Triethylamin als Eluent.

### 1.1.2 Syntheseweg 2

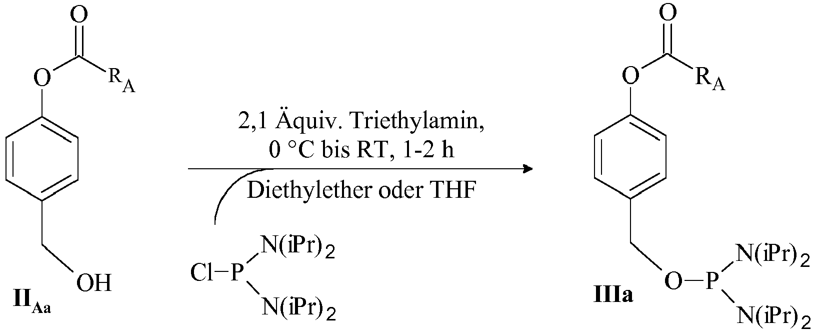

Unter Stickstoffatmosphäre werden 1 Äquivalent Bis(N,N-diisopropylamino)-chlorphosphin in Diethylether oder THF gelöst (12 mL/500 mg). Unter Eiskühlung wird eine Lösung aus 2,1 Äquivalenten Triethylamin und 1,9 Äquivalenten des Phenylesters **II_{Aa}** in Diethylether bzw. THF (entsprechend 5 mL) langsam zugetropft. Anschließend wird das Kühlbad entfernt und das Reaktionsgemisch bei Raumtemperatur gerührt. Nach ca. 1 bis 2 Stunden wird das entstandene Triethylammoniumchlorid durch Filtration und schließlich das Lösungsmittel unter vermindertem Druck entfernt. Ohne weitere Aufarbeitung erfolgt die Reinigung des Produktes **IIIa** am Chromatotron® mit Petrolether/Triethylamin als Eluent.

### 1.2 Synthese von Phosphoramiditen

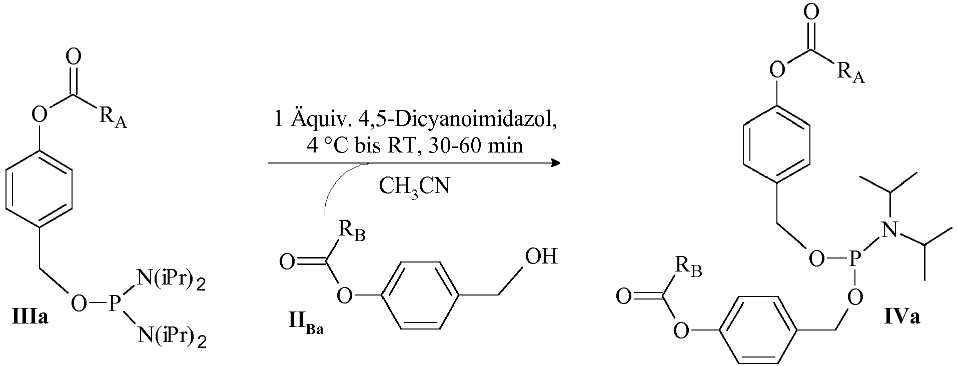

Unter Stickstoffatmosphäre werden 1,5 Äquivalente Phosphordiamidit **IIIa** zunächst mit Acetonitril coevaporiert und schließlich in 2 mL/100 mg gelöst. Bei Eiskühlung erfolgt die tropfenweise Zugabe von einer Lösung von 1 Äquivalent des entsprechenden 4-Acyloxybenzylalkohols **II_{Ba}** und 1 Äquivalent einer 0,25-molaren 4,5-Dicyanoimidazol-Aktivatorlösung in Acetonitril. Nach ca. 30 bis 60 min Rühren bei Raumtemperatur wird die Reaktion durch Entfernen des Lösungsmittels unter vermindertem Druck beendet. Der Rückstand wird in Petrolether/Triethylamin (9:1) aufgenommen und filtriert. Die Reinigung des Produkts **IVa** erfolgt am Chromatotron® mit Petrolether/Triethylamin als Eluent.

### 1.3 Synthese von DiPPro-, TriPPPro-Nukleotiden

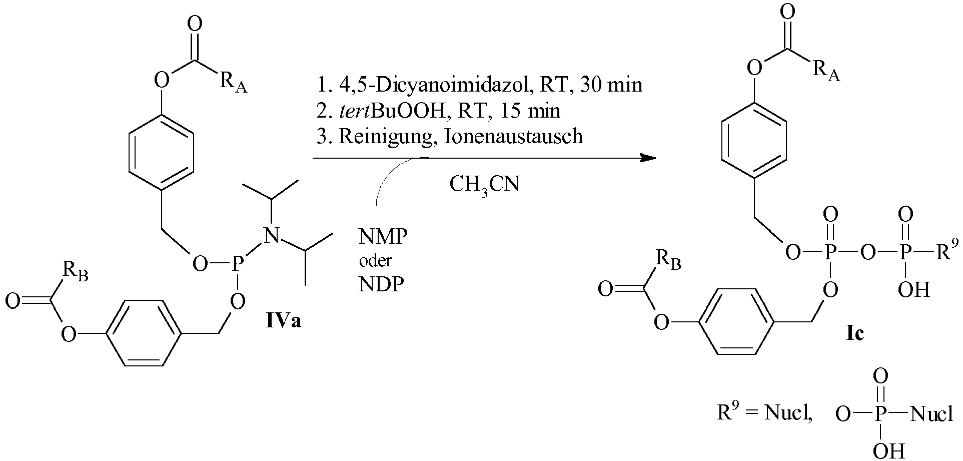

Die Reaktion wird unter Stickstoffatmosphäre durchgeführt. Es werden 1 Äquivalent des entsprechenden N(C₄H₉)₄⁺-Salzes des Nukleosidmonophosphats (NMP) oder Nukleosiddiphosphats (NDP) in Acetonitril gelöst (ca. 4 mL/100 g) und mit 1,5 Äquivalenten des zuvor mit Acetonitril coevaporierten Phosphoramidits **IVa** versetzt. Die Aktivierung des Phosphoramidits **IVa** erfolgt durch schrittweise Zugabe einer 0,25-molaren 4,5-Dicyanoimidazol-Aktivatorlösung (in Acetonitril). Zum Start werden 0,5 Äquivalente hinzugegeben und dann alle 5 min weitere 0,25 Äquivalente, bis ein vollständiger Umsatz erreicht ist (maximal 1,75 Äquivalente). Die Oxidation erfolgt durch Zugabe von 1,5 Äquivalenten einer 5,5molaren *t*BuOOH-Lösung in n-Decan. Umkehrphasen(RP)-Chromatographie ("Puri Flash"), gefolgt von einem Ionenaustausch zu NH₄⁺ und erneute, eventuell mehrfache, RP-Chromatographie (Puri Flash), liefern das Di*PP*ro-Nukleotid oder Tri*PPP*ro-Nukleotid **Ic** in Ausbeuten von bis zu 85%.

### 2. Herstellung asymmetrisch maskierter Nukleosiddi- und Nukleosidtriphosphat-Verbindungen über Phosphonatverbindungen

### 2.1 Herstellung einer asymmetrisch substituierten Phosphonatverbindung

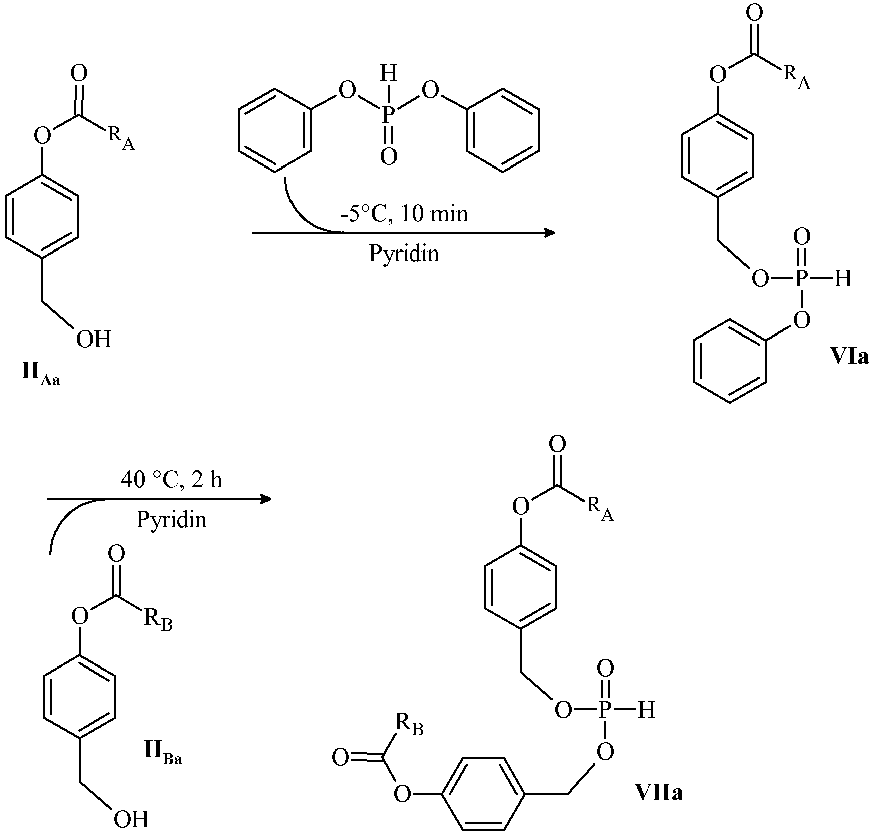

Zur Synthese der asymmetrisch substituierten Phosphonatverbindung **VIIa** wird unter Stickstoffatmosphäre 1 Äquivalent Phenylester **II_{Aa}** in Pyridin (ca. 15 mL/g IIA) langsam zu einer auf-5 °C gekühlten Lösung aus 1,3 Äquivalenten Diphenylphosphonat (DPP) in Pyridin (ca. 20 mL/g DPP) getropft. Nach zehnminütigem Rühren erfolgt die Zugabe von 1,6 Äquivalenten Phenylester **II_{Ba}** und die Lösung wird für 2 h bei 40 °C gerührt. Anschließend werden alle flüchtigen Bestandteile bei 40 °C im Hochvakuum entfernt. Außerdem wird der Rückstand zweimal mit Toluol coevaporiert. Die Reinigung des Produkts VIIa erfolgt durch Chromatographie mit Petrolether/Ethylacetat/Essigsäure als Eluent.

Die resultierende Phosphonatverbindung **VIIa** kann mit einem Nukleosidmonophosphat oder einem Analogon davon, in Reaktion gebracht werden, um ein Nukleosiddiphosphat oder Nukleosiddiphosphatanalogon (Di*PP*roNukleotid(analogon)) herzustellen.

Zur Herstellung von Nukleosidtriphosphaten (Tri*PPP*ro-Nukleotiden) werden zunächst Dibenzylpyrophosphatderivate der Verbindung **VIIa** und anschließend die Nukleosidtriphosphate hergestellt.

### 2.2 Herstellung von Dibenzylpyrophosphatderivaten der Verbindung VIIa

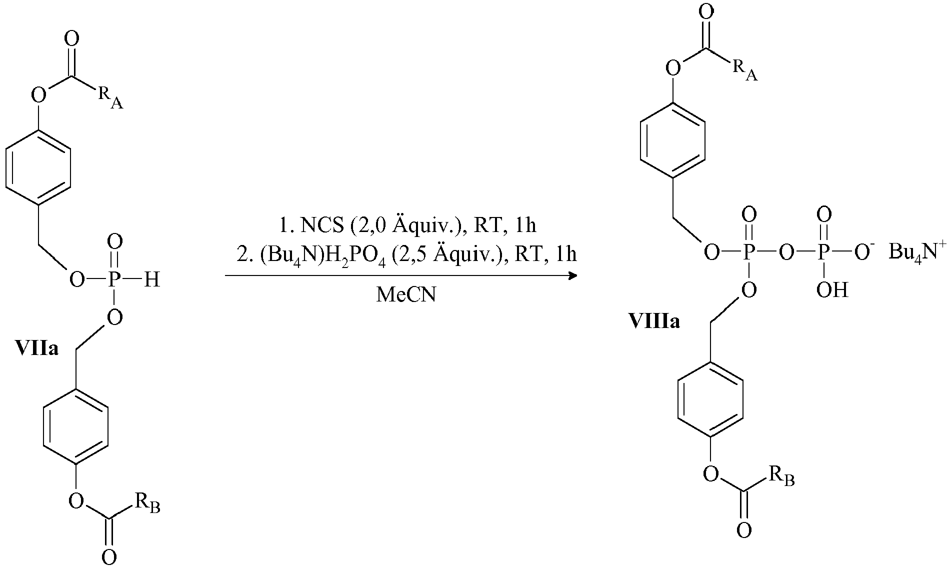

Unter Stickstoffatmosphäre werden 1 Äquivalent Phosphonat **VIIa** in Acetonitril (ca. 10 mL/g Phosphonat) falls notwendig unter leichtem Erwärmen gelöst und mit 2 Äquivalenten N-Chlorsuccinimid (NCS) versetzt. Nach einer Stunde wird die Lösung umgehend zu 2,5 Äquivalenten Tetra-*n*-butylammoniummonophosphat in Acetonitril (ca. 20 mL/g Phosphatsalz) getropft. Die Lösung wird 1 h Stunden gerührt und anschließend vom Lösungsmittel befreit. Der Rückstand wird in Dichlormethan und kalter 1 M Ammoniumacetat-Lösung aufgenommen und die Phasen unter Verwendung einer Zentrifuge getrennt. Die organische Phase wird ein weiteres Mal mit kaltem Wasser gewaschen, über Natriumsulfat getrocknet, filtriert und abschließend vom Lösungsmittel befreit. Das gewünschte Produkt **VIIIa** liegt daraufhin in sehr hoher Reinheit vor. Der Umsatz ist quantitativ.

### 2.3 Herstellung der TriPPPro-Nukleotide

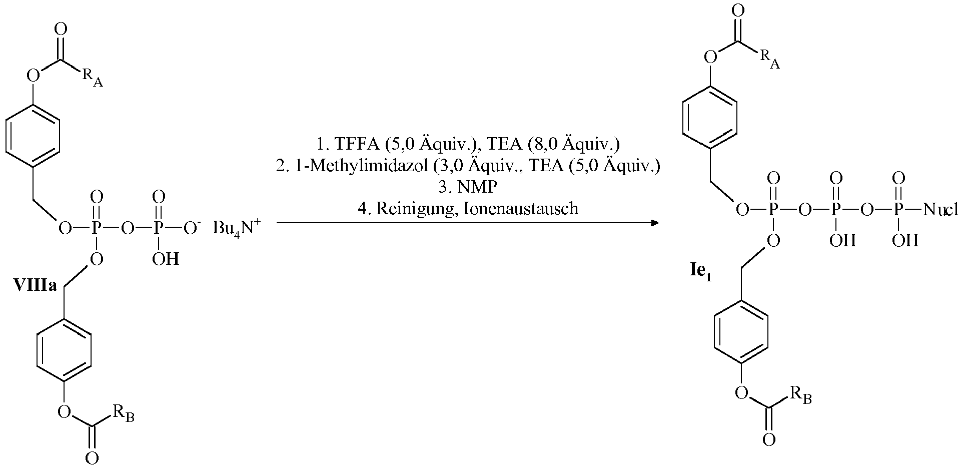

Die Reaktion wird unter Stickstoffatmosphäre durchgeführt. Bei 0 °C werden zunächst 5 Äquivalente Trifluoressigsäureanhydrid (TFAA) und 8 Äquivalente Triethylamin (TEA) in Acetonitril (ca. 20 mL/g TFAA) vermengt. Diese Lösung wird danach zu 1 Äquivalent Dibenzylpyrophosphatverbindung **VIIIa** in Acetonitril (ca. 20 mL/g Dibenzylpyrophosphat) gegeben. Nach 10 min bei 0 °C werden alle flüchtigen Bestandteile im Hochvakuum entfernt. Der Rückstand wird in Acetonitril (ca. 20 mL/g Dibenzylpyrophosphat) suspendiert und mit 5 Äquivalenten TEA und 3 Äquivalenten Methylimidazol umgesetzt. Nach fünfminütigem Rühren werden 1 Äquivalent des entsprechenden N(C₄H₉)₄⁺-Salzes des Nucleosidmonophosphats (NMP, Nucl = Nucleosid) in Acetonitril gelöst (ca. 20 mL/g NMP) hinzugegeben und die Reaktion nach 3 h durch Entfernen des Lösungsmittels unter vermindertem Druck beendet. Durch Umkehrphasen(RP)-Chromatographie ("Puri Flash"), gefolgt von einem Ionenaustausch zu NH₄⁺ und erneute, eventuell mehrfache, RP-Chromatographie, kann das Tri*PPP*ro-Nukleotid **Ie₁** in Ausbeuten von bis zu 50% isoliert werden.

### 3. Hydrolysestudien

Hydrolysestudien in CEM/0-Zellextrakt gaben Aufschluss über die enzymatische Freisetzung von Nukleosiddiphosphaten aus den Prodrugs. Dabei werden die Proben in Zellextrakt inkubiert und zu unterschiedlichen Zeitpunkten abgebrochen. Die Analyse der Hydrolyseproben erfolgte HPL-chromatographisch. Bei zwei unterschiedlichen Gruppen an den Acyleinheiten der Masken wird die Instabilere schnell gespalten (z.B. R = CH₃: t_{1/2} = ca. 2 min; R = C₄H₉: t_{1/2} = ca. 45 bis 60 min), wodurch die Bildung des stabileren Intermediats erfolgt. Anschließend hydrolysiert das Intermediat quantitativ zum Diphosphat. Die Bildung von d4TDP wurde durch Co-Injektion einer d4TDP-Lösung bestätigt.

Die chemische Stabilität der Prodrugs wurde auch in Phosphatpuffer bei pH 7,3 geprüft. Lösungen der Di*PP*ro- bzw. Tri*PPP*ro-Nucleotide werden hierzu mit einem Phosphatpuffer (PBS) nach Sörensen versetzt und die Hydrolyse HPL-chromatographisch verfolgt. Die Experimente zeigen, dass alle Prodrugs über eine erheblich höhere chemische als enzymatische Stabilität verfügen (z.B. Halbwertszeiten von CH₃/C₉H₁₉-Di*PP*ro-d4TDP (Abbau des Prodrugs): t_{1/2} (PBS) = 48 h, t_{1/2} (CEM/0) = 2 min). Bedingt durch die höhere Stabilität kann zu Beginn der Hydrolyse in Phosphatpuffer auch die Bildung des Nukleosidmonophosphats beobachtet werden. Da durch die Wahl einer asymmetrischen Maske aber eine Gruppe von geringerer Stabilität mit einer Gruppe von hoher Lipophilie kombiniert ist, bildet sich, wie auch im Zellextrakt, schnell das Intermediat (und zwar das stabilere), wodurch die Diphosphatbildung deutlich präferiert wird.

## Patentansprüche

1. Verbindung der allgemeinen Formel I 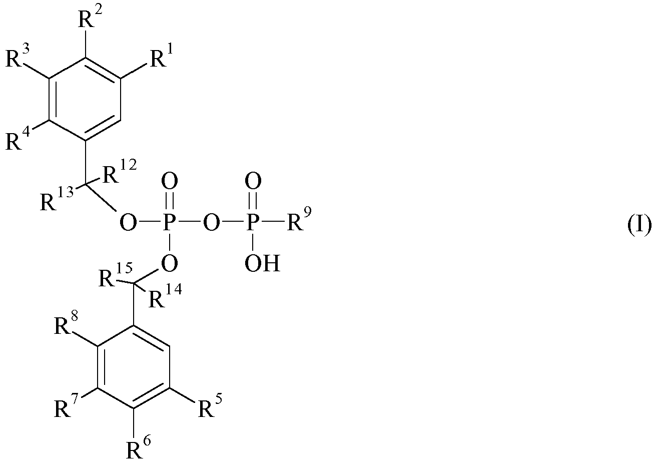 oder ein pharmazeutisch annehmbares Salz davon, wobei
R¹, R³, R⁵ und R⁷ unabhängig voneinander H, Halogen, NO₂, CN, SO₃H, ein cyclischer, acyclischer, linearer oder verzweigter aliphatischer oder heteroaliphatischer Rest, oder ein aromatischer oder heteroaromatischer Rest sind,
R² und R⁴ unabhängig voneinander H oder Z-C(Y)-R_{A}, jedoch nicht beide H sind,
R⁶ und R⁸ unabhängig voneinander H oder Z-C(Y)-R_{B}, jedoch nicht beide H sind,
Z, Y unabhängig voneinander O, S oder HN sind,
R_{A} und R_{B} verschieden und jeweils ein cyclischer, acyclischer, linearer oder verzweigter aliphatischer oder heteroaliphatischer Rest, oder ein aromatischer oder heteroaromatischer Rest sind,
R⁹ Nukleosid, Nukleosidmonophosphat, Nukleosidanalogon, Nukleosidmonophosphatanalogon, O-R¹⁰, OP(O)(OH)-R¹⁰ oder OP(O)(OH)O-R¹⁰ ist, wobei
R¹⁰ ein cyclischer, acyclischer, linearer oder verzweigter aliphatischer oder heteroaliphatischer Rest, oder ein aromatischer oder heteroaromatischer Rest ist,
R¹², R¹³, R¹⁴ und R¹⁵ unabhängig voneinander H, ein cyclischer, acyclischer, linearer oder verzweigter aliphatischer oder heteroaliphatischer Rest, ein aromatischer oder heteroaromatischer Rest, und/oder ein Elektronenakzeptor sind.

2. Verbindung nach Anspruch 1, wobei
a) R¹, R³, R⁵ und R⁷
i. jeweils unabhängig voneinander H, Halogen, NO₂, CN, SO₃H, ein cyclischer, acyclischer, linearer oder verzweigter C₁₋₂₀-Aliphatrest oder C₁₋₂₀-Heteroaliphatrest, oder ein C₅₋₂₀-Aromatenrest oder C₃₋₂₀-Heteroaromatenrest sind, oder
ii. jeweils unabhängig voneinander H, Halogen, NO₂, CN, SO₃H, ein cyclischer, acyclischer, linearer oder verzweigter C₁₋₁₀-Aliphatrest oder C₁₋₁₀-Heteroaliphatrest, oder ein C₅₋₁₂-Aromatenrest oder C₃₋₁₂-Heteroaromatenrest sind, oder
iii. jeweils unabhängig voneinander ausgewählt sind aus der Gruppe bestehend aus H, Halogen, NO₂, CN, SO₃H, C₁₋₂₀-Alkyl, C₂₋₂₀-Alkenyl, C₂₋₂₀-Alkinyl, C₄₋₂₀-Alkeninyl, C₃₋₂₀-Cycloalkyl, C₃₋₂₀-Cycloalkenyl, C₅₋₂₀-Cycloalkinyl, C₅₋₂₀-Cycloalkeninyl, C₁₋₂₀-Heteroalkyl, C₂₋₂₀-Heteroalkenyl, C₂₋₂₀-Heteroalkinyl, C₄₋₂₀-Heteroalkeninyl, C₅₋₂₄-Aryl, C₃₋₂₄-Heteroaryl, oder
iv. jeweils unabhängig voneinander ausgewählt sind aus der Gruppe bestehend aus H, Halogen, NO₂, CN, SO₃H, C₁₋₁₀-Alkyl, C₂₋₁₀-Alkenyl, C₂₋₁₀-Alkinyl, C₄₋₁₀-Alkeninyl, C₃₋₁₀-Cycloalkyl, C₃₋₁₀-Cycloalkenyl, C₅₋₁₀-Cycloalkinyl, C₅₋₁₀-Cycloalkeninyl, C₁₋₁₀-Heteroalkyl, C₂₋₁₀-Heteroalkenyl, C₂₋₁₀-Heteroalkinyl, C₄₋₁₀-Heteroalkeninyl, C₅₋₁₂-Aryl, C₃₋₁₂-Heteroaryl, oder
v. alle H, sind,
b) R_{A} und R_{B} verschieden und
i. jeweils unabhängig voneinander ein cyclischer, acyclischer, linearer oder verzweigter C₁₋₂₀-Aliphatrest oder C₁₋₂₀-Heteroaliphatrest, oder ein C₅₋₂₀-Aromatenrest oder C₃-₂₀-Heteroaromatenrest sind, oder
ii. jeweils unabhängig voneinander H, ein cyclischer, acyclischer, linearer oder verzweigter C₁₋₁₀-Aliphatrest oder C₁₋₁₀-Heteroaliphatrest, oder ein C₅₋₁₂-Aromatenrest oder C₃₋₁₂-Heteroaromatenrest sind, oder
iii. jeweils unabhängig voneinander ausgewählt sind aus der Gruppe bestehend aus C₁₋₂₀-Alkyl, C₂₋₂₀-Alkenyl, C₂₋₂₀-Alkinyl, C₄₋₂₀-Alkeninyl, C₃₋₂₀-Cycloalkyl, C₃₋₂₀-Cycloalkenyl, C₅₋₂₀-Cycloalkinyl, C₅₋₂₀-Cycloalkeninyl, C₁₋₂₀-Heteroalkyl, C₂₋₂₀-Heteroalkenyl, C₂₋₂₀-Heteroalkinyl, C₄₋₂₀-Heteroalkeninyl, C₅₋₂₄-Aryl, C₃₋₂₄-Heteroaryl, vorzugsweise C₁₋₂₀-Alkyl oder C₁₋₂₀-Alkenyl,
c) _{R}¹⁰
i. ein cyclischer, acyclischer, linearer oder verzweigter C₁₋₂₀-Aliphatrest oder C₁₋₂₀-Heteroaliphatrest, oder ein C₅₋₂₀-Aromatenrest oder C₃-₂₀-Heteroaromatenrest ist, oder
ii. ein cyclischer, acyclischer, linearer oder verzweigter C₁₋₁₀-Aliphatrest oder C₁₋₁₀-Heteroaliphatrest, oder ein C₅₋₁₂-Aromatenrest oder C₃₋₁₂-Heteroaromatenrest ist, oder
iii. C₁₋₂₀-Alkyl oder C₁₋₂₀-Alkenyl oder ein Zuckerrest ist, und
d) R¹², R¹³, R¹⁴ und R¹⁵
i. jeweils unabhängig voneinander H, ein cyclischer, acyclischer, linearer oder verzweigter C₁₋₂₀-Aliphatrest oder C₁₋₂₀-Heteroaliphatrest, ein C₅₋₂₀-Aromatenrest oder C₃₋₂₀-Heteroaromatenrest, und/oder ein Elektronenakzeptor sind, oder
ii. jeweils unabhängig voneinander H, ein cyclischer, acyclischer, linearer oder verzweigter C₁₋₁₀-Aliphatrest oder C₁₋₁₀-Heteroaliphatrest, oder ein C₅₋₁₂-Aromatenrest oder C₃₋₁₂-Heteroaromatenrest sind, oder
iii. jeweils unabhängig voneinander ausgewählt sind aus der Gruppe bestehend aus H, C₁₋₂₀-Alkyl, C₂₋₂₀-Alkenyl, C₂-₂₀-Alkinyl, C₄₋₂₀-Alkeninyl, C₃₋₂₀-Cycloalkyl, C₃₋₂₀-Cycloalkenyl, C₅₋₂₀-Cycloalkinyl, C₅₋₂₀-Cycloalkeninyl, C₁₋₂₀-Heteroalkyl, C₂₋₂₀-Heteroalkenyl, C₂₋₂₀-Heteroalkinyl, C₄₋₂₀-Heteroalkeninyl, C₅₋₂₄-Aryl, C₃₋₂₄-Heteroaryl, oder
iv. jeweils unabhängig voneinander ausgewählt sind aus der Gruppe bestehend aus H, C₁₋₁₀-Alkyl, C₂₋₁₀-Alkenyl, C₂₋₁₀-Alkinyl, C₄₋₁₀-Alkeninyl, C₃₋₁₀-Cycloalkyl, C₃₋₁₀-Cycloalkenyl, C₅₋₁₀-Cycloalkinyl, C₅₋₁₀-Cycloalkeninyl, C₁₋₁₀-Heteroalkyl, C₂₋₁₀-Heteroalkenyl, C₂₋₁₀-Heteroalkinyl, C₄₋₁₀-Heteroalkeninyl, C₅₋₁₂-Aryl, C₃₋₁₂-Heteroaryl, oder
v. alle H, sind, oder
vi. ein Elektronenakzeptor oder H sind, mit der Maßgabe, dass R¹² und R¹⁴ jeweils H und R¹³ und R¹⁵ jeweils ein Elektronenakzeptor, oder R¹³ und R¹⁵ jeweils H und R¹² und R¹⁴ jeweils ein Elektronenakzeptor sind.

3. Verbindung nach Anspruch 1 oder 2, wobei die Verbindung I eine Verbindung gemäß der folgenden Formel Id 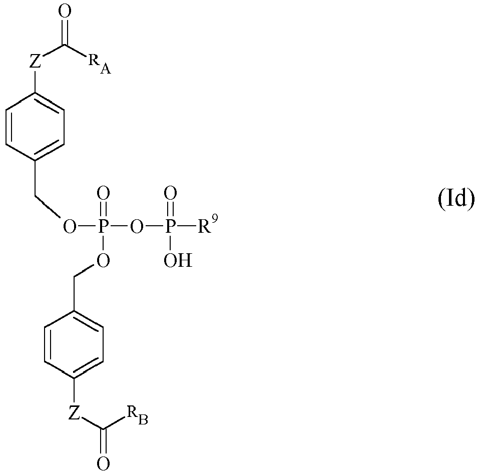 oder ein pharmazeutisch annehmbares Salz davon ist, und wobei Z = O, S oder HN, vorzugsweise O ist.

4. Verbindung nach einem der Ansprüche 1 bis 3 zur Verwendung als Arzneimittel.

5. Verbindung nach einem der Ansprüche 1 bis 3 zur Verwendung als antivirales Arzneimittel, vorzugsweise als antiretrovirales Arzneimittel, Arzneimittel zur Behandlung einer HIV-Infektion, Hepatitis-Infektion, von Influenza oder hämorrhagischem Fieber.

6. Pharmazeutische Zusammensetzung, umfassend eine Verbindung nach einem der Ansprüche 1 bis 3 und einen pharmazeutisch annehmbaren Träger.

7. Verfahren zur Herstellung einer Verbindung der allgemeinen Formel I 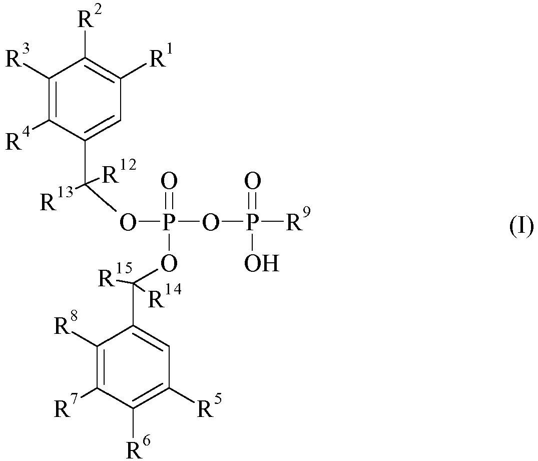 oder eines pharmazeutisch annehmbaren Salzes davon, wobei
R¹, R³, R⁵ und R⁷ unabhängig voneinander H, Halogen, NO₂, CN, SO₃H, oder ein substituierter oder unsubstituierter cyclischer, acyclischer, linearer oder verzweigter aliphatischer oder heteroaliphatischer Rest, oder ein substituierter oder unsubstituierter aromatischer oder heteroaromatischer Rest sind,
R² und R⁴ unabhängig voneinander H oder Z-C(Y)-R_{A}, jedoch nicht beide H sind,
R⁶ und R⁸ unabhängig voneinander H, Z-C(Y)-R_{B}, jedoch nicht beide H sind,
Z, Y unabhängig voneinander O, S oder HN sind,
R_{A} und R_{B} verschieden und jeweils ein substituierter oder unsubstituierter cyclischer, acyclischer, linearer oder verzweigter aliphatischer oder heteroaliphatischer Rest, oder ein substituierter oder unsubstituierter aromatischer oder heteroaromatischer Rest sind,
R⁹ Nukleosid, Nukleosidmonophosphat, Nukleosidanalogon, Nukleosidmonophosphatanalogon, O-R¹⁰, OP(O)(OH)-R¹⁰ oder OP(O)(OH)O-R¹⁰ ist, wobei
R¹⁰ ein substituierter oder unsubstituierter cyclischer, acyclischer, linearer oder verzweigter aliphatischer oder heteroaliphatischer Rest, oder ein substituierter oder unsubstituierter aromatischer oder heteroaromatischer Rest ist,
R¹², R¹³, R¹⁴ und R¹⁵ unabhängig voneinander H, ein substituierter oder unsubstituierter cyclischer, acyclischer, linearer oder verzweigter aliphatischer oder heteroaliphatischer Rest, ein substituierter oder unsubstituierter aromatischer oder heteroaromatischer Rest, und/oder ein Elektronenakzeptor sind,
umfassend die folgenden Schritte:
a₁) Inreaktionbringen einer Verbindung der allgemeinen Formel II_{A} 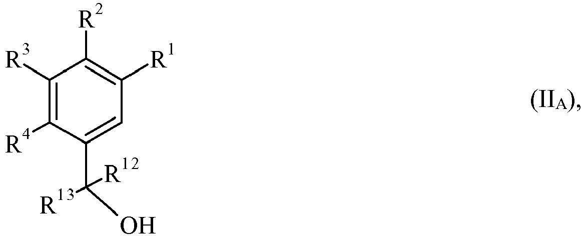 wobei R¹, R², R³, R⁴, R¹² und R¹³ wie oben definiert sind,
mit i) Phosphortrichlorid PCl3 und N,N-Di*iso*propylamin, oder ii) Bis(N,N-di*iso*propylamino)chlorphosphin
zur Synthese einer Verbindung der allgemeinen Formel III 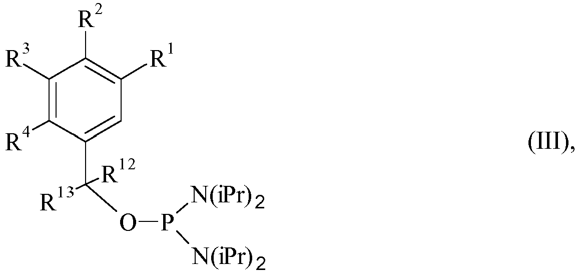
b₁) Inreaktionbringen der in a) erhaltenen Verbindung der Formel III mit einer Verbindung der allgemeinen Formel II_{B} 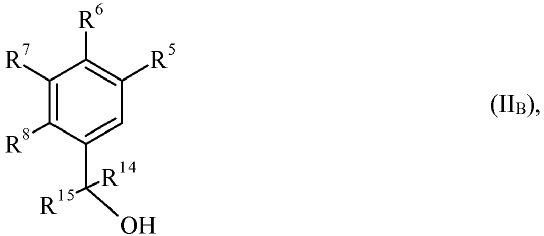 wobei R⁵, R⁶, R⁷ R⁸, R¹⁴ und R¹⁵ wie oben definiert sind,
zur Synthese einer Verbindung der allgemeinen Formel IV 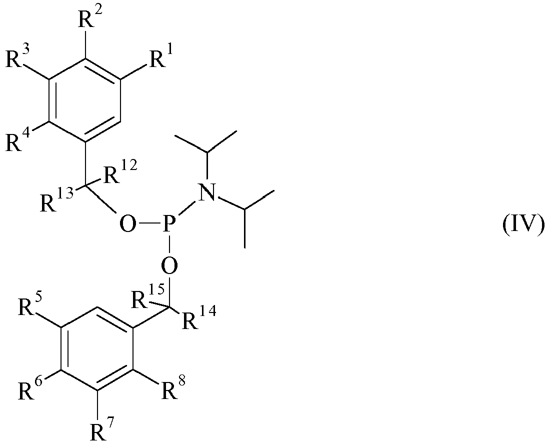
c₁) Inreaktionbringen der in b) erhaltenen Verbindung der Formel IV mit einer Verbindung gemäß der allgemeinen Formel V 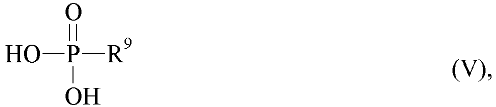 wobei R⁹ wie oben definiert ist,
oder
a₂) Inreaktionbringen einer Verbindung der allgemeinen Formel II_{A} 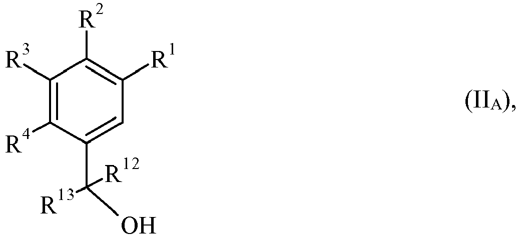 wobei R¹, R², R³ R⁴, R¹² und R¹³ wie oben definiert sind,
mit Diphenylphosphonat zur Synthese einer Verbindung der allgemeinen Formel VI 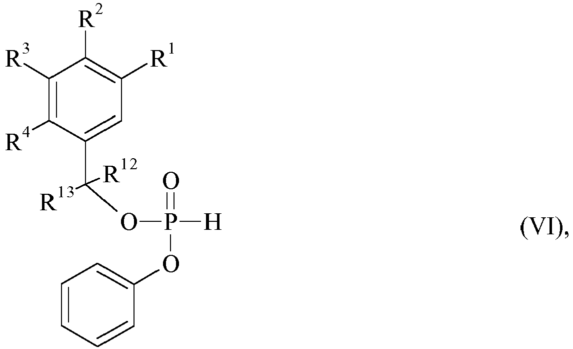
b₂) Inreaktionbringen der in a) erhaltenen Verbindung der Formel VI mit einer Verbindung der allgemeinen Formel II_{B} 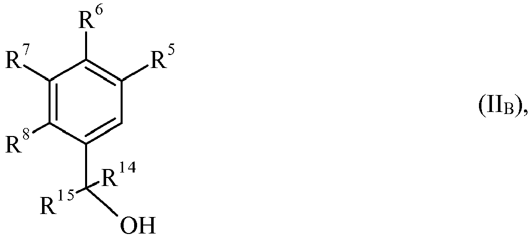 wobei R⁵, R⁶, R⁷ R⁸, R¹⁴ und R¹⁵ wie oben definiert sind,
zur Synthese einer Verbindung der allgemeinen Formel VII 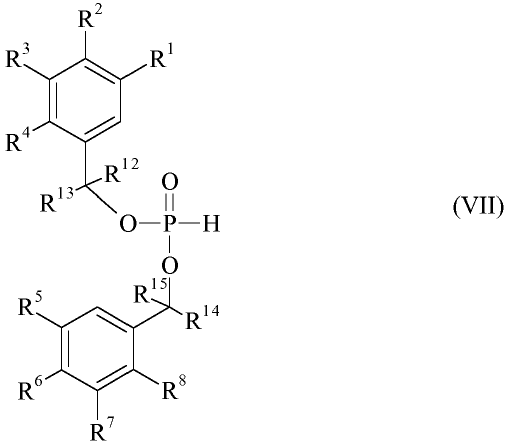
c₂) Inreaktionbringen der in b) erhaltenen Verbindung der Formel VII mit i) einer Verbindung gemäß der allgemeinen Formel V 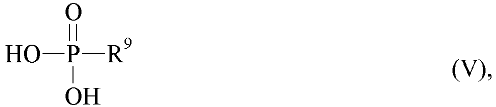 oder ii) mit Phosphorsäure oder einem Phosphorsäuresalz und R⁹ oder einer R⁹ umfassenden Verbindung,
wobei R⁹ wie oben definiert ist.

8. Verfahren nach Anspruch 7, wobei
a) R¹, R³, R⁵ und R⁷
i. jeweils unabhängig voneinander H, Halogen, NO₂, CN, SO₃H, ein substituierter oder unsubstituierter cyclischer, acyclischer, linearer oder verzweigter C₁₋₂₀-Aliphatrest oder C₁₋₂₀-Heteroaliphatrest, oder ein substituierter oder unsubstituierter C₅₋₂₀-Aromatenrest oder C₃₋₂₀-Heteroaromatenrest sind, oder
ii. jeweils unabhängig voneinander H, Halogen, NO₂, CN, SO₃H, ein substituierter oder unsubstituierter cyclischer, acyclischer, linearer oder verzweigter C₁₋₁₀-Aliphatrest oder C₁₋₁₀-Heteroaliphatrest, oder ein substituierter oder unsubstituierter C₅₋₁₂-Aromatenrest oder C₃₋₁₂-Heteroaromatenrest sind, oder
iii. jeweils unabhängig voneinander ausgewählt sind aus der Gruppe bestehend aus H, Halogen, NO₂, CN, SO₃H, substituiertem oder unsubstituiertem C₁₋₂₀-Alkyl, substituiertem oder unsubstituiertem C₂₋₂₀-Alkenyl, substituiertem oder unsubstituiertem C₂-₂₀-Alkinyl, substituiertem oder unsubstituiertem C₄₋₂₀-Alkeninyl, substituiertem oder unsubstituiertem C₃₋₂₀-Cycloalkyl, substituiertem oder unsubstituiertem C₃₋₂₀-Cycloalkenyl, substituiertem oder unsubstituiertem C₅₋₂₀-Cycloalkinyl, substituiertem oder unsubstituiertem C₅₋₂₀-Cycloalkeninyl, substituiertem oder unsubstituiertem C₁₋₂₀-Heteroalkyl, substituiertem oder unsubstituiertem C₂₋₂₀-Heteroalkenyl, substituiertem oder unsubstituiertem C₂₋₂₀-Heteroalkinyl, substituiertem oder unsubstituiertem C₄₋₂₀-Heteroalkeninyl, substituiertem oder unsubstituiertem C₅₋₂₄-Aryl, substituiertem oder unsubstituiertem C₃₋₂₄-Heteroaryl, oder
iv. jeweils unabhängig voneinander ausgewählt sind aus der Gruppe bestehend aus H, Halogen, NO₂, CN, SO₃H, substituiertem oder unsubstituiertem C₁₋₁₀-Alkyl, substituiertem oder unsubstituiertem C₂₋₁₀-Alkenyl, substituiertem oder unsubstituiertem C₂₋₁₀-Alkinyl, substituiertem oder unsubstituiertem C₄₋₁₀-Alkeninyl, substituiertem oder unsubstituiertem C₃₋₁₀-Cycloalkyl, substituiertem oder unsubstituiertem C₃₋₁₀-Cycloalkenyl, substituiertem oder unsubstituiertem C₅₋₁₀-Cycloalkinyl, substituiertem oder unsubstituiertem C₅₋₁₀-Cycloalkeninyl, substituiertem oder unsubstituiertem C₁₋₁₀-Heteroalkyl, substituiertem oder unsubstituiertem C₂₋₁₀-Heteroalkenyl, substituiertem oder unsubstituiertem C₂₋₁₀-Heteroalkinyl, substituiertem oder unsubstituiertem C₄₋₁₀-Heteroalkeninyl, substituiertem oder unsubstituiertem C₅₋₁₂-Aryl, substituiertem oder unsubstituiertem C₃₋₁₂-Heteroaryl, oder
v. alle H, sind,
b) R_{A} und R_{B} verschieden und
i. jeweils unabhängig voneinander ein substituierter oder unsubstituierter cyclischer, acyclischer, linearer oder verzweigter C₁₋₂₀-Aliphatrest oder C₁₋₂₀-Heteroaliphatrest, oder ein substituierter oder unsubstituierter C₅₋₂₀-Aromatenrest oder C₃-₂₀-Heteroaromatenrest sind, oder
ii. jeweils unabhängig voneinander ein substituierter oder unsubstituierter cyclischer, acyclischer, linearer oder verzweigter C₁₋₁₀-Aliphatrest oder C₁₋₁₀-Heteroaliphatrest, oder ein substituierter oder unsubstituierter C₅₋₁₂-Aromatenrest oder C₃₋₁₂-Heteroaromatenrest sind, oder
iii. jeweils unabhängig voneinander ausgewählt sind aus der Gruppe bestehend aus substituiertem oder unsubstituiertem C₁₋₂₀-Alkyl, substituiertem oder unsubstituiertem C₂₋₂₀-Alkenyl, substituiertem oder unsubstituiertem C₂-₂₀-Alkinyl, substituiertem oder unsubstituiertem C₄₋₂₀-Alkeninyl, substituiertem oder unsubstituiertem C₃₋₂₀-Cycloalkyl, substituiertem oder unsubstituiertem C₃₋₂₀-Cycloalkenyl, substituiertem oder unsubstituiertem C₅₋₂₀-Cycloalkinyl, substituiertem oder unsubstituiertem C₅₋₂₀-Cycloalkeninyl, substituiertem oder unsubstituiertem C₁₋₂₀-Heteroalkyl, substituiertem oder unsubstituiertem C₂₋₂₀-Heteroalkenyl, substituiertem oder unsubstituiertem C₂₋₂₀-Heteroalkinyl, substituiertem oder unsubstituiertem C₄₋₂₀-Heteroalkeninyl, substituiertem oder unsubstituiertem C₅₋₂₄-Aryl, substituiertem oder unsubstituiertem C₃₋₂₄-Heteroaryl, vorzugsweise C₁₋₂₀-Alkyl oder C₁₋₂₀-Alkenyl, sind,
c) R¹⁰
i. ein substituierter oder unsubstituierter cyclischer, acyclischer, linearer oder verzweigter C₁₋₂₀-Aliphatrest oder C₁₋₂₀-Heteroaliphatrest, oder ein substituierter oder unsubstituierter C₅₋₂₀-Aromatenrest oder C₃-₂₀-Heteroaromatenrest ist, oder
ii. ein substituierter oder unsubstituierter cyclischer, acyclischer, linearer oder verzweigter C₁₋₁₀-Aliphatrest oder C₁₋₁₀-Heteroaliphatrest, oder ein substituierter oder unsubstituierter C₅₋₁₂-Aromatenrest oder C₃₋₁₂-Heteroaromatenrest ist, oder
iii. C₁₋₂₀-Alkyl oder C₁₋₂₀-Alkenyl oder ein Zuckerrest ist,
und
d) R¹², R¹³, R¹⁴ und R¹⁵
i. jeweils unabhängig voneinander H, ein substituierter oder unsubstituierter cyclischer, acyclischer, linearer oder verzweigter C₁₋₂₀-Aliphatrest oder C₁₋₂₀-Heteroaliphatrest, ein substituierter oder unsubstituierter C₅₋₂₀-Aromatenrest oder C₃₋₂₀-Heteroaromatenrest, und/oder ein Elektronenakzeptor sind, oder
ii. jeweils unabhängig voneinander H, ein substituierter oder unsubstituierter cyclischer, acyclischer, linearer oder verzweigter C₁₋₁₀-Aliphatrest oder C₁₋₁₀-Heteroaliphatrest, oder ein substituierter oder unsubstituierter C₅₋₁₂-Aromatenrest oder C₃₋₁₂-Heteroaromatenrest sind, oder
iii. jeweils unabhängig voneinander ausgewählt sind aus der Gruppe bestehend aus H, substituiertem oder unsubstituiertem C₁₋₂₀-Alkyl, substituiertem oder unsubstituiertem C₂₋₂₀-Alkenyl, substituiertem oder unsubstituiertem C₂-₂₀-Alkinyl, substituiertem oder unsubstituiertem C₄₋₂₀-Alkeninyl, substituiertem oder unsubstituiertem C₃₋₂₀-Cycloalkyl, substituiertem oder unsubstituiertem C₃₋₂₀-Cycloalkenyl, substituiertem oder unsubstituiertem C₅₋₂₀-Cycloalkinyl, substituiertem oder unsubstituiertem C₅₋₂₀-Cycloalkeninyl, substituiertem oder unsubstituiertem C₁₋₂₀-Heteroalkyl, substituiertem oder unsubstituiertem C₂₋₂₀-Heteroalkenyl, substituiertem oder unsubstituiertem C₂₋₂₀-Heteroalkinyl, substituiertem oder unsubstituiertem C₄₋₂₀-Heteroalkeninyl, substituiertem oder unsubstituiertem C₅₋₂₄-Aryl, substituiertem oder unsubstituiertem C₃₋₂₄-Heteroaryl, oder
iv. jeweils unabhängig voneinander ausgewählt sind aus der Gruppe bestehend aus H, substituiertem oder unsubstituiertem C₁₋₁₀-Alkyl, substituiertem oder unsubstituiertem C₂₋₁₀-Alkenyl, substituiertem oder unsubstituiertem C₂₋₁₀-Alkinyl, substituiertem oder unsubstituiertem C₄₋₁₀-Alkeninyl, substituiertem oder unsubstituiertem C₃₋₁₀-Cycloalkyl, substituiertem oder unsubstituiertem C₃₋₁₀-Cycloalkenyl, substituiertem oder unsubstituiertem C₅₋₁₀-Cycloalkinyl, substituiertem oder unsubstituiertem C₅₋₁₀-Cycloalkeninyl, substituiertem oder unsubstituiertem C₁₋₁₀-Heteroalkyl, substituiertem oder unsubstituiertem C₂₋₁₀-Heteroalkenyl, substituiertem oder unsubstituiertem C₂₋₁₀-Heteroalkinyl, substituiertem oder unsubstituiertem C₄₋₁₀-Heteroalkeninyl, substituiertem oder unsubstituiertem C₅₋₁₂-Aryl, substituiertem oder unsubstituiertem C₃₋₁₂-Heteroaryl, oder
v. alle H, sind, oder
vi. ein Elektronenakzeptor oder H sind, mit der Maßgabe, dass R¹² und R¹⁴ jeweils H und R¹³ und R¹⁵ jeweils ein Elektronenakzeptor, oder R¹³ und R¹⁵ jeweils H und R¹² und R¹⁴ jeweils ein Elektronenakzeptor sind.

## Claims

1. Compound having general formula I 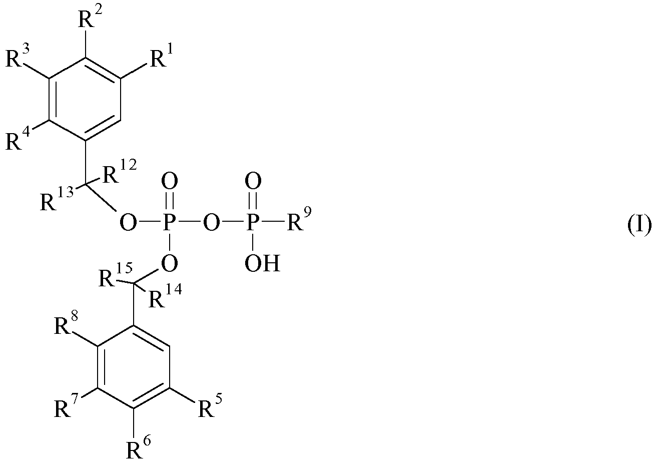 or a pharmaceutically acceptable salt thereof, wherein
R¹, R³, R⁵ and R⁷ are independently H, halogen, NO₂, CN, SO₃H, a cyclic, acyclic, linear or branched aliphatic or heteroaliphatic radical, or an aromatic or heteroaromatic radical,
R² and R⁴ are independently H or Z-C(Y)-R_{A}, but are not both H,
R⁶ and R⁸ are independently H, Z-C(Y)-R_{B}, but are not both H,
Z, Y is independently O, S or HN,
R_{A} and R_{B} are different and each is a cyclic, acyclic, linear or branched aliphatic or heteroaliphatic radical, or an aromatic or heteroaromatic radical,
R⁹ is nucleoside, nucleoside monophosphate, nucleoside analogue, nucleoside monophosphate analogue, O-R¹⁰, OP(O)(OH)-R¹⁰ or OP(O)(OH)-O-R¹⁰, wherein R¹⁰ is a cyclic, acyclic, linear or branched aliphatic or heteroaliphatic radical, or an aromatic or heteroaromatic radical,
R¹², R¹³, R¹⁴ and R¹⁵ are independently H, a cyclic, acyclic, linear or branched aliphatic or heteroaliphatic radical, an aromatic or heteroaromatic radical, and/or an electron acceptor.

2. Compound according to claim 1, wherein
a) R¹, R³, R⁵ and R⁷
i. are each independently H, halogen, NO₂, CN, SO₃H, a cyclic, acyclic, linear or branched C₁₋₂₀-aliphatic radical or C₁₋₂₀-heteroaliphatic radical, or a C₅₋₂₀-aromatic radical or C₃₋₂₀-heteroaromatic radical, or
ii. are each independently H, halogen, NO₂, CN, SO₃H, a cyclic, acyclic, linear or branched C₁₋₁₀-aliphatic radical or C₁₋₁₀-heteroaliphatic radical, or a C₅₋₁₂-aromatic radical or C₃₋₁₂-heteroaromatic radical, or
iii. are each independently selected from the group consisting of H, halogen, NO₂, CN, SO₃H, C₁₋₂₀-alkyl, C₂₋₂₀-alkenyl, C₂₋₂₀-alkinyl, C₄₋₂₀-alkeninyl, C₃₋₂₀-cycloalkyl, C₃₋₂₀-cycloalkenyl, C₅₋₂₀-cycloalkinyl, C₅₋₂₀-cycloalkeninyl, C₁₋₂₀-heteroalkyl, C₂₋₂₀-heteroalkenyl, C₂₋₂₀-heteroalkinyl, C₄₋₂₀-heteroalkeninyl, C₅₋₂₄-aryl, C₃₋₂₄-heteroaryl, or
iv. are each independently selected from the group consisting of H, halogen, NO₂, CN, SO₃H, C₁₋₁₀-alkyl, C₂₋₁₀-alkenyl, C₂₋₁₀-alkinyl, C₄₋₁₀-alkeninyl, C₃₋₁₀-cycloalkyl, C₃₋₁₀-cycloalkenyl, C₅₋₁₀-cycloalkinyl, C₅₋₁₀-cycloalkeninyl, C₁₋₁₀-heteroalkyl, C₂₋₁₀-heteroalkenyl, C₂₋₁₀-heteroalkinyl, C₄₋₁₀-heteroalkeninyl, C₅₋₁₂-aryl, C₃₋₁₂-heteroaryl, or
v. are all H,
b) R_{A} and R_{B} are different and
i. are each independently a cyclic, acyclic, linear or branched C₁₋₂₀-aliphatic radical or C₁₋₂₀-heteroaliphatic radical, or a C₅₋₂₀-aromatic radical or C₃₋₂₀-heteroaromatic radical, or
ii. are each independently H, a cyclic, acyclic, linear or branched C₁₋₁₀-aliphatic radical or C₁₋₁₀-heteroaliphatic radical, or a C₅₋₁₂-aromatic radical or C₃₋₁₂-heteroaromatic radical, or
iii. are each independently selected from the group consisting of C₁₋₂₀-alkyl, C₂₋₂₀-alkenyl, C₂₋₂₀-alkinyl, C₄₋₂₀-alkeninyl, C₃₋₂₀-cycloalkyl, C₃₋₂₀-cycloalkenyl, C₅₋₂₀-cycloalkinyl, C₅₋₂₀-cycloalkeninyl, C₁₋₂₀-heteroalkyl, C₂₋₂₀-heteroalkenyl, C₂₋₂₀-heteroalkinyl, C₄₋₂₀-heteroalkeninyl, C₅₋₂₄-aryl, C₃₋₂₄-heteroaryl, preferably C₁₋₂₀-Alkyl or C₁₋₂₀-alkenyl,
c) _{R}¹⁰
i. is a cyclic, acyclic, linear or branched C₁₋₂₀-aliphatic radical or C₁₋₂₀-heteroaliphatic radical, or a C₅₋₂₀-aromatic radical or C₃₋₂₀-heteroaromatic radical, or
ii. is a cyclic, acyclic, linear or branched C₁₋₁₀-aliphatic radical or C₁₋₁₀-heteroaliphatic radical, or a C₅₋₁₂-aromatic radical or C₃₋₁₂-heteroaromatic radical, or
iii. is a C₁₋₂₀-alkyl or C₁₋₂₀-alkenyl or a sugar radical,
and
d) R¹², R¹³, R¹⁴ and R¹⁵
i. are each independently H, a cyclic, acyclic, linear or branched C₁₋₂₀-aliphatic radical or C₁₋₂₀-heteroaliphatic radical, a C₅₋₂₀-aromatic radical or C₃₋₂₀-heteroaromatic radical, and/or an electron acceptor, or
ii. are each independently H, a cyclic, acyclic, linear or branched C₁₋₁₀-aliphatic radical or C₁₋₁₀-heteroaliphatic radical, or a C₅₋₁₂-aromatic radical or C₃₋₁₂-heteroaromatic radical, or
iii. are each independently selected from the group consisting of H, C₁₋₂₀-alkyl, C₂₋₂₀-alkenyl, C₂₋₂₀-alkinyl, C₄₋₂₀-alkeninyl, C₃₋₂₀-cycloalkyl, C₃₋₂₀-cycloalkenyl, C₅₋₂₀-cycloalkinyl, C₅₋₂₀-cycloalkeninyl, C₁₋₂₀-heteroalkyl, C₂₋₂₀-heteroalkenyl, C₂₋₂₀-heteroalkinyl, C₄₋₂₀-heteroalkeninyl, C₅₋₂₄-aryl, C₃₋₂₄-heteroaryl, or
iv. are each independently selected from the group consisting of H, C₁₋₁₀-alkyl, C₂₋₁₀-alkenyl, C₂₋₁₀-alkinyl, C₄₋₁₀-alkeninyl, C₃₋₁₀-cycloalkyl, C₃₋₁₀-cycloalkenyl,C₅₋₁₀-cycloalkinyl, C₅₋₁₀-cycloalkeninyl, C₁₋₁₀-heteroalkyl, C₂₋₁₀-heteroalkenyl, C₂₋₁₀-heteroalkinyl, C₄₋₁₀-heteroalkeninyl, C₅₋₁₂-aryl, C₃₋₁₂-heteroaryl, or
v. are all H, or
vi. are an electron acceptor or H, with the proviso that R¹² and R¹⁴ are each H and R¹³ and R¹⁵ are each an electron acceptor, or R¹³ and R¹⁵ are each H and R¹² and R¹⁴ are each an electron acceptor.

3. Compound according to claim 1 or 2, wherein compound I is a compound according to the following formula Id 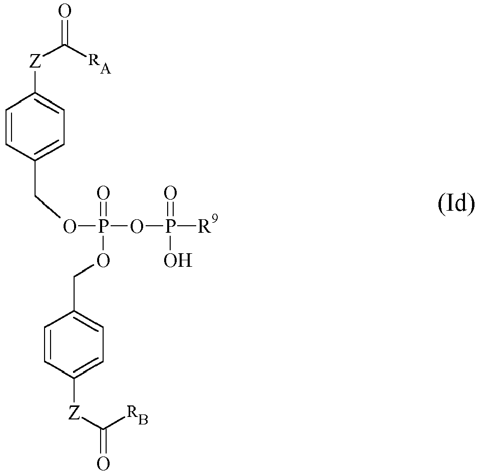 or is a pharmaceutically acceptable salt thereof, and wherein Z is = O, S or HN, preferably O.

4. Compound according to any one of claims 1 to 3 for use as a medicinal product.

5. Compound according to any one of claims 1 to 3 for use as an antiviral medicinal product, preferably as antiretroviral medicinal product, medicinal product for the treatment of an HIV-infection, hepatitis-infection, influenza or haemorrhagic fever.

6. Pharmaceutical composition, comprising a compound according to any one of claims 1 to 3 and a pharmaceutically acceptable carrier.

7. Method for producing a compound having general formula I 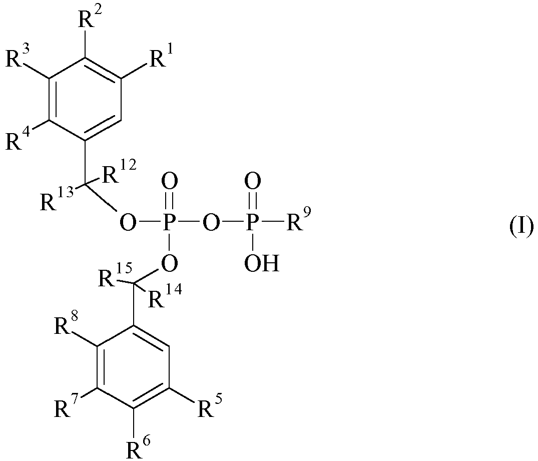 or a pharmaceutically acceptable salt thereof, wherein
R¹, R³, R⁵ and R⁷ are independently H, halogen, NO₂, CN, SO₃H, or a substituted or unsubstituted cyclic, acyclic, linear or branched aliphatic or heteroaliphatic radical, or a substituted or unsubstituted aromatic or heteroaromatic radical,
R² and R⁴ are independently H or Z-C(Y)-R_{A}, but are not both H,
R⁶ and R⁸ are independently H, Z-C(Y)-R_{B}, but are not both H,
Z, Y is independently O, S or HN,
R_{A} and R_{B} are different and each is a substituted or unsubstituted cyclic, acyclic, linear or branched aliphatic or heteroaliphatic radical, or a substituted or unsubstituted aromatic or heteroaromatic radical,
R⁹ is nucleoside, nucleoside monophosphate, nucleoside analogue, nucleoside monophosphate analogue, O-R¹⁰, OP(O)(OH)-R¹⁰ or OP(O)(OH)-O-R¹⁰, wherein
R¹⁰ is a substituted or unsubstituted cyclic, acyclic, linear or branched aliphatic or heteroaliphatic radical, or a substituted or unsubstituted aromatic or heteroaromatic radical, and wherein
R¹², R¹³, R¹⁴ and R¹⁵ are independently H, a substituted or unsubstituted cyclic, acyclic, linear or branched aliphatic or heteroaliphatic radical, a substituted or unsubstituted aromatic or heteroaromatic radical, and/or an electron acceptor,
comprising the following steps:
a₁) reacting a compound having general formula II_{A} 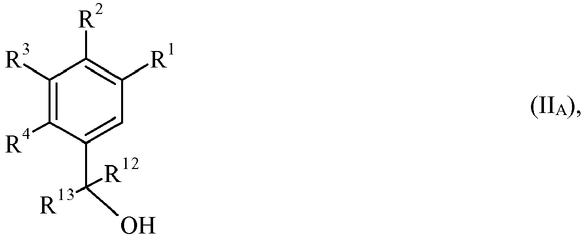 wherein R¹, R², R³, R⁴, R¹² and R¹³ are defined as above,
with i) phosphorus trichloride PCl₃ and *N,N*-di*iso*propylamine, or ii) bis(N,N-di*iso*propylamino)chlorophosphine
to synthesise a compound having general formula III 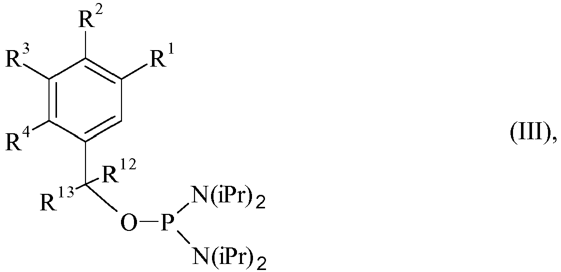
b₁) reacting the compound having formula III obtained in a) with a compound having general formula II_{B} 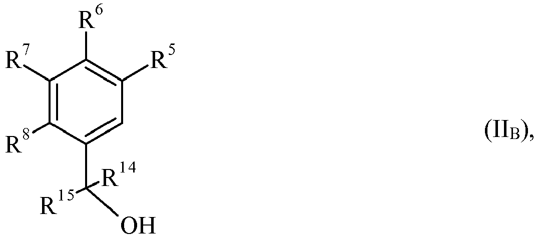 wherein R⁵, R⁶, R⁷, R⁸, R¹⁴ and R¹⁵ are defined as above, to synthesise a compound having general formula IV 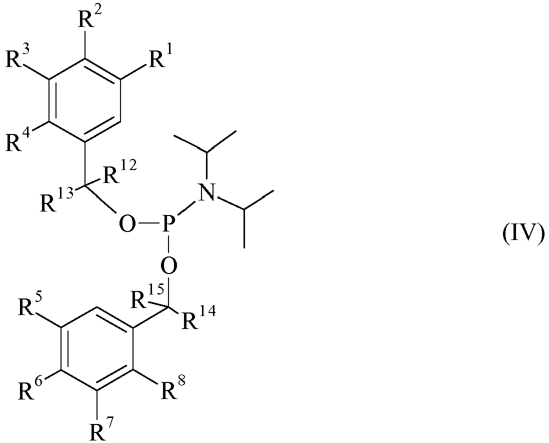
c₁) reacting the compound having formula IV obtained in b) to react with a compound according to general formula V 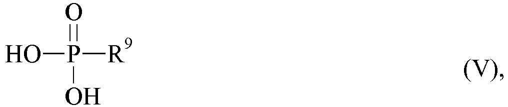 wherein R⁹ is defined as above,
or
a₂) reacting a compound having general formula II_{A} 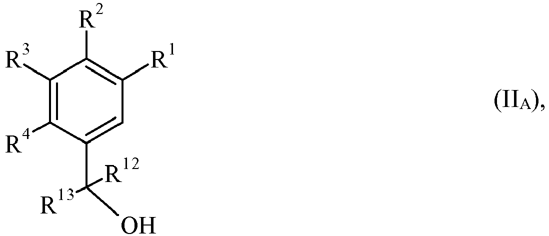 wherein R¹, R², R³, R⁴, R¹², and R¹³ are defined as above,
with diphenylphosphonate to synthesise a compound having general VI 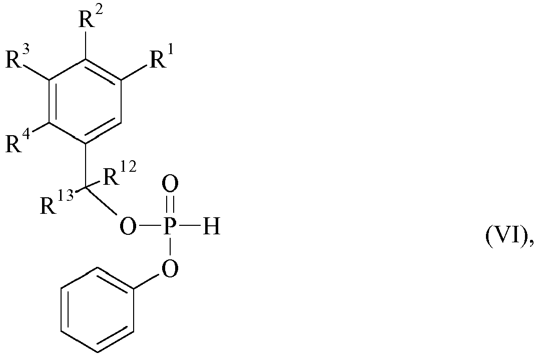
b₂) reacting the compound with formula VI obtained in a) with a compound having general formula II_{B} 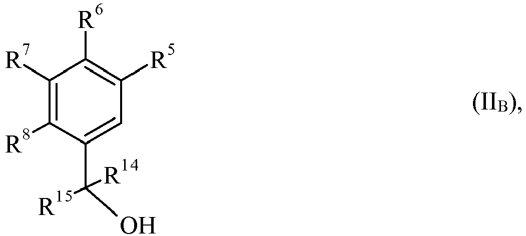 wherein R⁵, R⁶, R⁷, R⁸, R¹⁴ and R¹⁵ are defined as above,
to synthesise a compound having general formula VII 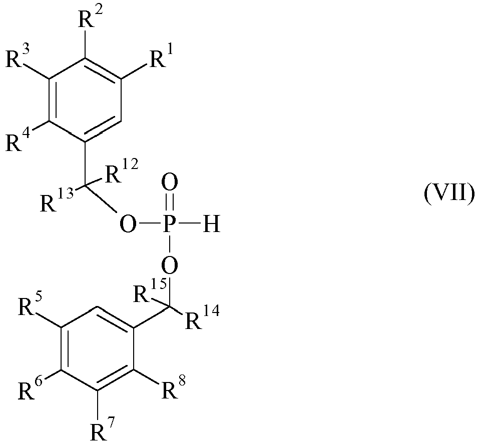
c₂) reacting the compound with formula VII obtained in b) with i) a compound according to general formula V 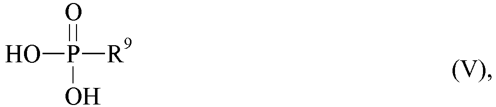 or ii) with phosphoric acid or a phosphoric acid salt and R⁹ or a compound comprising R⁹,
wherein R⁹ is defined as above.

8. Method according to claim 7, wherein
a) R¹, R³, R⁵ and R⁷
i. are each independently H, halogen, NO₂, CN, SO₃H, a substituted or unsubstituted cyclic, acyclic, linear or branched C₁₋₂₀-aliphatic radical or C₁₋₂₀-heteroaliphatic radical, or a substituted or unsubstituted C₅₋₂₀-aromatic radical or C₃₋₂₀-heteroaromatic radical, or
ii. are each independently H, halogen, NO₂, CN, SO₃H, a substituted or unsubstituted cyclic, acyclic, linear or branched C₁₋₁₀-aliphatic radical or C₁₋₁₀-heteroaliphatic radical, or a substituted or unsubstituted C₅₋₁₂-aromatic radical or C₃₋₁₂-heteroaromatic radical, or
iii. are each independently selected from the group consisting of H, halogen, NO₂, CN, SO₃H, substituted or unsubstituted C₁₋₂₀-alkyl, substituted or unsubstituted C₂₋₂₀-alkenyl, substituted or unsubstituted C₂₋₂₀-alkinyl, substituted or unsubstituted C₄₋₂₀-alkeninyl, substituted or unsubstituted C₃₋₂₀-cycloalkyl, substituted or unsubstituted C₃₋₂₀-cycloalkenyl, substituted or unsubstituted C₅₋₂₀-cycloalkinyl, substituted or unsubstituted C₅₋₂₀-cycloalkeninyl, substituted or unsubstituted C₁₋₂₀-heteroalkyl, substituted or unsubstituted C₂₋₂₀-heteroalkenyl, substituted or unsubstituted C₂₋₂₀-heteroalkinyl, substituted or unsubstituted C₄₋₂₀-heteroalkeninyl, substituted or unsubstituted C₅₋₂₄-aryl, substituted or unsubstituted C₃₋₂₄-heteroaryl, or
iv. are each independently selected from the group consisting of H, halogen, NO₂, CN, SO₃H, substituted or unsubstituted C₁₋₁₀-alkyl, substituted or unsubstituted C₂₋₁₀-alkenyl, substituted or unsubstituted C₂₋₁₀-alkinyl, substituted or unsubstituted C₄₋₁₀-alkeninyl, substituted or unsubstituted C₃₋₁₀-cycloalkyl, substituted or unsubstituted C₃₋₁₀-cycloalkenyl, substituted or unsubstituted C₅₋₁₀-cycloalkinyl, substituted or unsubstituted C₅₋₁₀-cycloalkeninyl, substituted or unsubstituted C₁₋₁₀-heteroalkyl, substituted or unsubstituted C₂₋₁₀-heteroalkenyl, substituted or unsubstituted C₂₋₁₀-heteroalkinyl, substituted or unsubstituted C₄₋₁₀-heteroalkeninyl, substituted or unsubstituted C₅₋₁₂-aryl, substituted or unsubstituted C₃₋₁₂-heteroaryl, or
v. are all H,
b) R_{A} and R_{B} are different and
i. are each independently a substituted or unsubstituted cyclic, acyclic, linear or branched C₁₋₂₀-aliphatic radical or C₁₋₂₀-heteroaliphatic radical, or a substituted or unsubstituted C₅₋₂₀-aromatic radical or C₃₋₂₀-heteroaromatic radical, or
ii. are each independently H, a substituted or unsubstituted cyclic, acyclic, linear or branched C₁₋₁₀-aliphatic radical or C₁₋₁₀-heteroaliphatic radical, or a substituted or unsubstituted C₅₋₁₂-aromatic radical or C₃₋₁₂-heteroaromatic radical, or
iii. are each independently selected from the group consisting of substituted or unsubstituted C₁₋₂₀-alkyl, substituted or unsubstituted C₂₋₂₀-alkenyl, substituted or unsubstituted C₂₋₂₀-alkinyl, substituted or unsubstituted C₄₋₂₀-alkeninyl, substituted or unsubstituted C₃₋₂₀-cycloalkyl, substituted or unsubstituted C₃₋₂₀-cycloalkenyl, substituted or unsubstituted C₅₋₂₀-cycloalkinyl, substituted or unsubstituted C₅₋₂₀-cycloalkeninyl, substituted or unsubstituted C₁₋₂₀-heteroalkyl, substituted or unsubstituted C₂₋₂₀-heteroalkenyl, substituted or unsubstituted C₂₋₂₀-heteroalkinyl, substituted or unsubstituted C₄₋₂₀-heteroalkeninyl, substituted or unsubstituted C₅₋₂₄-aryl, substituted or unsubstituted C₃₋₂₄-heteroaryl, preferably C₁₋₂₀-Alkyl or C₁₋₂₀-alkenyl,
c) _{R}¹⁰
i. is a substituted or unsubstituted cyclic, acyclic, linear or branched C₁₋₂₀-aliphatic radical or C₁₋₂₀-heteroaliphatic radical, or a substituted or unsubstituted C₅₋₂₀-aromatic radical or C₃₋₂₀-heteroaromatic radical, or
ii. is a substituted or unsubstituted cyclic, acyclic, linear or branched C₁₋₁₀-aliphatic radical or C₁₋₁₀-heteroaliphatic radical, or a substituted or unsubstituted C₅₋₁₂-aromatic radical or C₃₋₁₂-heteroaromatic radical, or
iii. is a C₁₋₂₀-alkyl or C₁₋₂₀-alkenyl or a sugar radical,
and
d) R¹², R¹³, R¹⁴ and R¹⁵
i. are each independently H, a substituted or unsubstituted cyclic, acyclic, linear or branched C₁₋₂₀-aliphatic radical or C₁₋₂₀-heteroaliphatic radical, a substituted or unsubstituted C₅₋₂₀-aromatic radical or C₃₋₂₀-heteroaromatic radical, and/or an electron acceptor, or
ii. are each independently H, a substituted or unsubstituted cyclic, acyclic, linear or branched C₁₋₁₀-aliphatic radical or C₁₋₁₀-heteroaliphatic radical, or a substituted or unsubstituted C₅₋₁₂-aromatic radical or C₃₋₁₂-heteroaromatic radical, or
iii. are each independently selected from the group consisting of H, substituted or unsubstituted C₁₋₂₀-alkyl, substituted or unsubstituted C₂₋₂₀-alkenyl, substituted or unsubstituted C₂₋₂₀-alkinyl, substituted or unsubstituted C₄₋₂₀-alkeninyl, substituted or unsubstituted C₃₋₂₀-cycloalkyl, substituted or unsubstituted C₃₋₂₀-cycloalkenyl, substituted or unsubstituted C₅₋₂₀-cycloalkinyl, substituted or unsubstituted C₅₋₂₀-cycloalkeninyl, substituted or unsubstituted C₁₋₂₀-heteroalkyl, substituted or unsubstituted C₂₋₂₀-heteroalkenyl, substituted or unsubstituted C₂₋₂₀-heteroalkinyl, substituted or unsubstituted C₄₋₂₀-heteroalkeninyl, substituted or unsubstituted C₅₋₂₄-aryl, substituted or unsubstituted C₃₋₂₄-heteroaryl, or
iv. are each independently selected from the group consisting of H, substituted or unsubstituted C₁₋₁₀-alkyl, substituted or unsubstituted C₂₋₁₀-alkenyl, substituted or unsubstituted C₂₋₁₀-alkinyl, substituted or unsubstituted C₄₋₁₀-alkeninyl, substituted or unsubstituted C₃₋₁₀-cycloalkyl, substituted or unsubstituted C₃₋₁₀-cycloalkenyl,substituted or unsubstituted C₅₋₁₀-cycloalkinyl, substituted or unsubstituted C₅₋₁₀-cycloalkeninyl, substituted or unsubstituted C₁₋₁₀-heteroalkyl, substituted or unsubstituted C₂₋₁₀-heteroalkenyl, substituted or unsubstituted C₂₋₁₀-heteroalkinyl, substituted or unsubstituted C₄₋₁₀-heteroalkeninyl, substituted or unsubstituted C₅₋₁₂-aryl, substituted or unsubstituted C₃₋₁₂-heteroaryl, or
v. are all H, or
vi. are an electron acceptor or H, providing that R¹² and R¹⁴ are each H and R¹³ and R¹⁵ are each an electron acceptor, or R¹³ and R¹⁵ are each H and R¹² and R¹⁴ are each an electron acceptor.

## Revendications

1. Composé répondant à la formule générale I 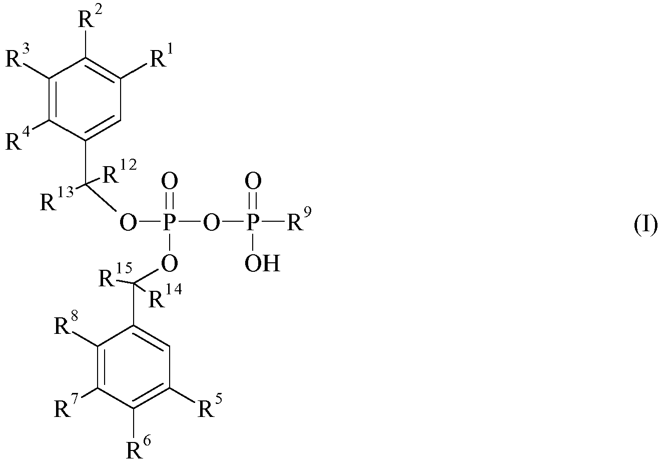 ou un sel pharmaceutiquement acceptable de ce dernier,
R¹, R³, R⁵ et R⁷ étant, indépendamment l'un de l'autre, H, halogène, NO₂, CN, SO₃H, un radical cyclique, acyclique, linéaire ou ramifié de nature aliphatique ou hétéroaliphatique, ou un radical aromatique ou hétéroaromatique,
R² et R⁴ étant, indépendamment l'un de l'autre, H ou Z-C(Y)-R_{A}, sans être H tous les deux,
R⁶ et R⁸ étant, indépendamment l'un de l'autre, H ou Z-C(Y)-R_{B}, sans être H tous les deux,
Z,Y étant, indépendamment l'un de l'autre, O, S ou HN,
R_{A} et R_{B} étant différents et représentant chacun un radical cyclique, acyclique, linéaire ou ramifié de nature aliphatique ou hétéroaliphatique, ou un radical aromatique ou hétéroaromatique,
R⁹ étant nucléoside, nucléoside-monophosphate, analogon de nucléoside, analogon de nucléoside-monophosphate, O-R¹⁰, OP(O)(OH)-R¹⁰ ou OP(O)(OH)O-R¹⁰, R¹⁰ étant un radical cyclique, acyclique, linéaire ou ramifié de nature aliphatique ou hétéroaliphatique, ou un radical aromatique ou hétéroaromatique,
R¹², R¹³, R¹⁴ et R¹⁵ étant, indépendamment l'un de l'autre, H, un radical cyclique, acyclique, linéaire ou ramifié de nature aliphatique ou hétéroaliphatique, ou un radical aromatique ou hétéroaromatique, et/ou un accepteur d'électrons.

2. Composé selon la revendication 1,
a) R¹, R³, R⁵ et R⁷
i. étant, indépendamment l'un de l'autre, H, halogène, NO₂, CN, SO₃H, un radical cyclique, acyclique, linéaire ou ramifié de nature C₁₋₂₀-aliphatique ou C₁₋₂₀-hétéroaliphatique, ou un radical C₅₋₂₀-aromatique ou C₃₋₂₀-hétéroaromatique, ou
ii. étant, indépendamment l'un de l'autre, H, halogène, NO₂, CN, SO₃H, un radical cyclique, acyclique, linéaire ou ramifié de nature C₁₋₁₀-aliphatique ou C₁₋₁₀-hétéroaliphatique, ou un radical C₅₋₁₂-aromatique ou C₃₋₁₂-hétéroaromatique, ou
iii. étant choisis, indépendamment l'un de l'autre, dans le groupe constitué de H, halogène, NO₂, CN, SO₃H, C₁₋₂₀-alkyle, C₂₋₂₀-alcényle, C₂₋₂₀-alcynyle, C₄₋₂₀-alcéninyle, C₃₋₂₀-cycloalkyle, C₃₋₂₀-cycloalcényle, C₅₋₂₀-cycloalcynyle, C₅₋₂₀-cycloalcéninyle, C₁₋₂₀-hétéroalkyle, C₂₋₂₀-hétéroalcényle, C₂₋₂₀-hétéroalcynyle, C₄₋₂₀-hétéroalcéninyle, C₅₋₂₄-aryle, C₃₋₂₄-hétéroaryle, ou
iv. étant choisis, indépendamment l'un de l'autre, dans le groupe constitué de H, halogène, NO₂, CN, SO₃H, C₁₋₁₀-alkyle, C₂₋₁₀-alcényle, C₂₋₁₀-alcynyle, C₄₋₁₀-alcéninyle, C₃₋₁₀-cycloalkyle, C₃₋₁₀-cycloalcényle, C₅₋₁₀-cycloalcynyle, C₅₋₁₀-cycloalcéninyle, C₁₋₁₀-hétéroalkyle, C₂₋₁₀-hétéroalcényle, C₂₋₁₀-hétéroalcynyle, C₄₋₁₀-hétéroalcéninyle, C₅₋₁₂-aryle, C₃₋₁₂-hétéroaryle, ou
v. étant tous H,
b) R_{A} et R_{B} étant différents et
i. étant, indépendamment l'un de l'autre, un radical cyclique, acyclique, linéaire ou ramifié de nature C₁₋₂₀-aliphatique ou C₁₋₂₀-hétéroaliphatique, ou un radical C₅₋₂₀-aromatique ou C₃₋₂₀-hétéroaromatique, ou
ii. étant, indépendamment l'un de l'autre, H, un radical cyclique, acyclique, linéaire ou ramifié de nature C₁₋₁₀-aliphatique ou C₁₋₁₀-hétéroaliphatique, ou un radical C₅₋₁₂-aromatique ou C₃₋₁₂-hétéroaromatique, ou
iii. étant choisis, indépendamment l'un de l'autre, dans le groupe constitué de C₁₋₂₀-alkyle, C₂₋₂₀-alcényle, C₂₋₂₀-alcynyle, C₄₋₂₀-alcéninyle, C₃₋₂₀-cycloalkyle, C₃₋₂₀-cycloalcényle, C₅₋₂₀-cycloalcynyle, C₅₋₂₀-cycloalcéninyle, C₁₋₂₀-hétéroalkyle, C₂₋₂₀-hétéroalcényle, C₂₋₂₀-hétéroalcynyle, C₄₋₂₀-hétéroalcéninyle, C₅₋₂₄-aryle, C₃₋₂₄-hétéroaryle, s'agissant préférentiellement de C₁₋₂₀-alkyle ou C₁₋₂₀-alcényle,
c) R¹⁰
i. étant un radical cyclique, acyclique, linéaire ou ramifié de nature C₁₋₂₀-aliphatique ou C₁₋₂₀-hétéroaliphatique, ou un radical C₅₋₂₀-aromatique ou C₃₋₂₀-hétéroaromatique, ou
ii. étant un radical cyclique, acyclique, linéaire ou ramifié de nature C₁₋₁₀-aliphatique ou C₁₋₁₀-hétéroaliphatique, ou un radical C₅₋₁₂-aromatique ou C₃₋₁₂-hétéroaromatique, ou
iii. étant C₁₋₂₀-alkyle ou C₁₋₂₀-alcényle ou un radical de sucre,
et
d) R¹², R¹³, R¹⁴ et R¹⁵
i. étant, indépendamment l'un de l'autre, H, un radical cyclique, acyclique, linéaire ou ramifié de nature C₁₋₂₀-aliphatique ou C₁₋₂₀-hétéroaliphatique, ou un radical C₅₋₂₀-aromatique ou C₃₋₂₀-hétéroaromatique, et/ou un accepteur d'électrons, ou
ii. étant, indépendamment l'un de l'autre, H, un radical cyclique, acyclique, linéaire ou ramifié de nature C₁₋₁₀-aliphatique ou C₁₋₁₀-hétéroaliphatique, ou un radical C₅₋₁₂-aromatique ou C₃₋₁₂-hétéroaromatique, ou
iii. étant choisis, indépendamment l'un de l'autre, dans le groupe constitué de H, C₁₋₂₀-alkyle, C₂₋₂₀-alcényle, C₂₋₂₀-alcynyle, C₄₋₂₀-alcéninyle, C₃₋₂₀-cycloalkyle, C₃₋₂₀-cycloalcényle, C₅₋₂₀-cycloalcynyle, C₅₋₂₀-cycloalcéninyle, C₁₋₂₀-hétéroalkyle, C₂₋₂₀-hétéroalcényle, C₂₋₂₀-hétéroalcynyle, C₄₋₂₀-hétéroalcéninyle, C₅₋₂₄-aryle, C₃₋₂₄-hétéroaryle, ou
iv. étant choisis, indépendamment l'un de l'autre, dans le groupe constitué de H, C₁₋₁₀-alkyle, C₂₋₁₀-alcényle, C₂₋₁₀-alcynyle, C₄₋₁₀-alcéninyle, C₃₋₁₀-cycloalkyle, C₃₋₁₀-cycloalcényle, C₅₋₁₀-cycloalcynyle, C₅₋₁₀-cycloalcéninyle, C₁₋₁₀-hétéroalkyle, C₂₋₁₀-hétéroalcényle, C₂₋₁₀-hétéroalcynyle, C₄₋₁₀-hétéroalcéninyle, C₅₋₁₂-aryle, C₃₋₁₂-hétéroaryle, ou
v. étant tous H, ou
vi. étant un accepteur d'électrons ou H, à condition que R¹² et R¹⁴ représentent chacun H, et R¹³ et R¹⁵ représentent chacun un accepteur d'électrons, ou que R¹³ et R¹⁵ représentent chacun H, et R¹² et R¹⁴ représentent chacun un accepteur d'électrons.

3. Composé selon les revendications 1 ou 2, le composé I étant un composé répondant à la formule Id 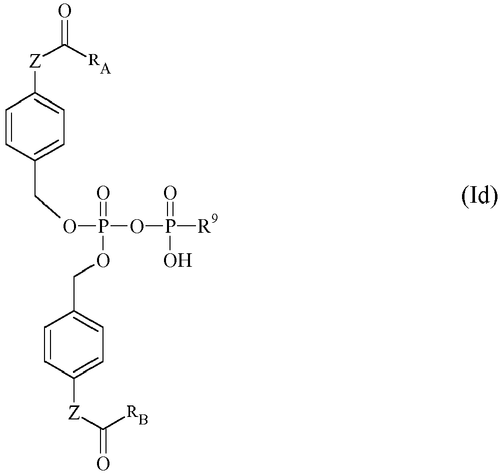 ou un sel pharmaceutiquement acceptable de ce dernier, et Z étant égal à O, S ou HN, de préférence à O.

4. Composé selon l'une des revendications 1 à 3, destiné à être utilisé en tant que médicament.

5. Composé selon l'une des revendications 1 à 3, destiné à être utilisé en tant que médicament antiviral, de préférence en tant que médicament antirétroviral, médicament destiné à traiter une infection par le VIH, une infection de type hépatite, une influenza ou une fièvre hémorragique.

6. Composition pharmaceutique comprenant un composé selon l'une des revendications 1 à 3 et un excipient pharmaceutiquement acceptable.

7. Procédé de fabrication d'un composé répondant à la formule générale I 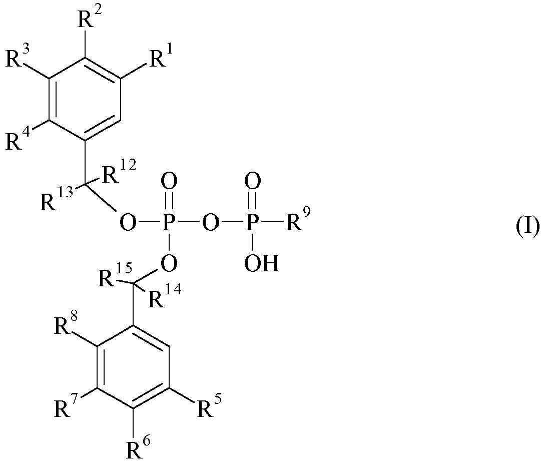 ou d'un sel pharmaceutiquement acceptable de ce dernier,
R₁, R₃, R₅ et R₇ étant, indépendamment l'un de l'autre, H, halogène, NO₂, CN, SO₃H, ou un radical cyclique, acyclique, linéaire ou ramifié, substitué ou non-substitué, de nature aliphatique ou hétéroaliphatique, ou un radical, substitué ou non-substitué, de nature aromatique ou hétéroaromatique,
R₂ et R₄ étant, indépendamment l'un de l'autre, H ou Z-C(Y)-R_{A}, sans être H tous les deux,
R⁶ et R⁸ étant, indépendamment l'un de l'autre, H, Z-C(Y)-R_{B}, sans être H tous les deux,
Z,Y étant, indépendamment l'un de l'autre, O, S ou HN,
R_{A} et R_{B} étant différents et représentant chacun un radical cyclique, acyclique, linéaire ou ramifié, substitué ou non-substitué, de nature aliphatique ou hétéroaliphatique, ou un radical, substitué ou non-substitué, de nature aromatique ou hétéroaromatique,
R⁹ étant nucléoside, nucléoside-monophosphate, analogon de nucléoside, analogon de nucléoside-monophosphate, O-R¹⁰, OP(O)(OH)-R¹⁰ ou OP(O)(OH)O-R¹⁰, R¹⁰ étant un radical cyclique, acyclique, linéaire ou ramifié, substitué ou non-substitué, de nature aliphatique ou hétéroaliphatique, ou un radical, substitué ou non-substitué, de nature aromatique ou hétéroaromatique,
R¹², R¹³, R¹⁴ et R¹⁵ étant, indépendamment l'un de l'autre, H, un radical cyclique, acyclique, linéaire ou ramifié, substitué ou non-substitué, de nature aliphatique ou hétéroaliphatique, ou un radical, substitué ou non-substitué, de nature aromatique ou hétéroaromatique, et/ou un accepteur d'électrons,
comprenant les étapes suivantes :
a₁) mise en réaction d'un composé répondant à la formule générale II_{A} 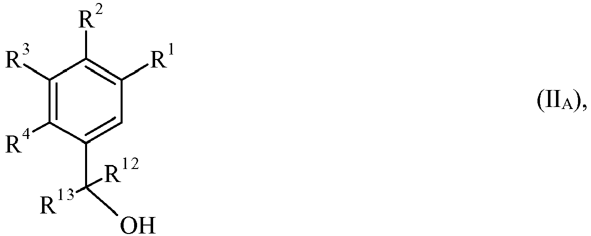 dans laquelle R¹, R², R³, R⁴, R¹² et R¹³ correspondent aux définitions indiquées ci-dessus,
avec i) du trichlorure de phosphore PCl₃ et de la N,N-di*iso*propylamine, ou ii) de la bis-(N,N-di*iso*propylamino)chlorophosphine
afin de synthétiser un composé répondant à la formule générale III 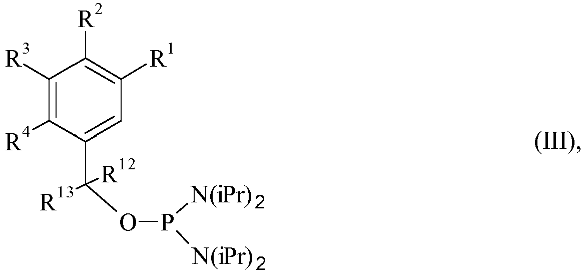
b₁) mise en réaction du composé obtenu sous a) de formule III avec un composé répondant à la formule générale II_{B} 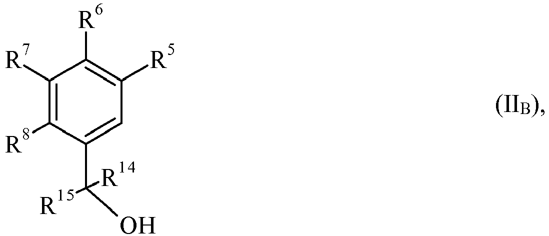 dans laquelle R⁵, R⁶, R⁷, R⁸, R¹⁴ et R¹⁵ correspondent aux définitions indiquées ci-dessus,
afin de synthétiser un composé répondant à la formule générale IV 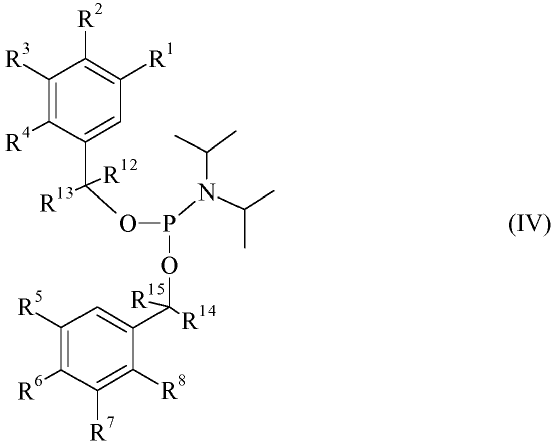
c₁) mise en réaction du composé obtenu sous b) de formule IV avec un composé répondant à la formule générale V 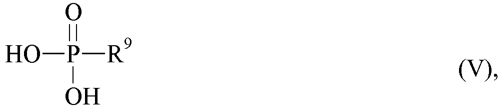 dans laquelle R⁹ correspond à la définition indiquée ci-dessus,
ou
a₂) mise en réaction d'un composé répondant à la formule générale II_{A} 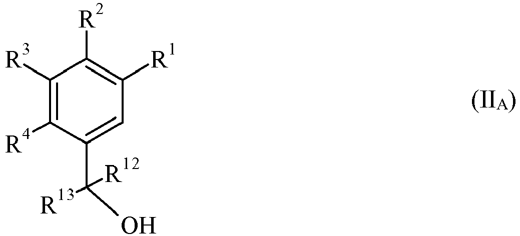 dans laquelle R¹, R², R³, R⁴, R¹² et R¹³ correspondent aux définitions indiquées ci-dessus,
avec du phosphonate de diphényle afin de synthétiser un composé répondant à la formule générale VI 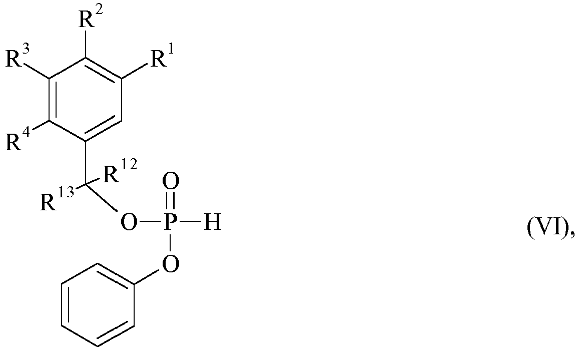
b₂) mise en réaction du composé obtenu sous a) de formule VI avec un composé répondant à la formule générale II_{B} 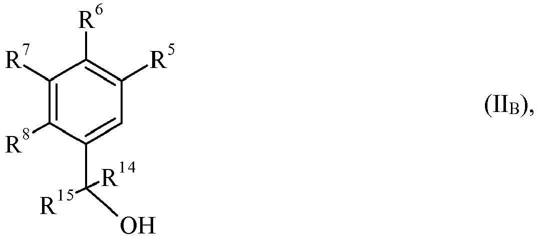 dans laquelle R⁵, R⁶, R⁷, R⁸, R¹⁴ et R¹⁵ correspondent aux définitions indiquées ci-dessus,
afin de synthétiser un composé répondant à la formule générale VII 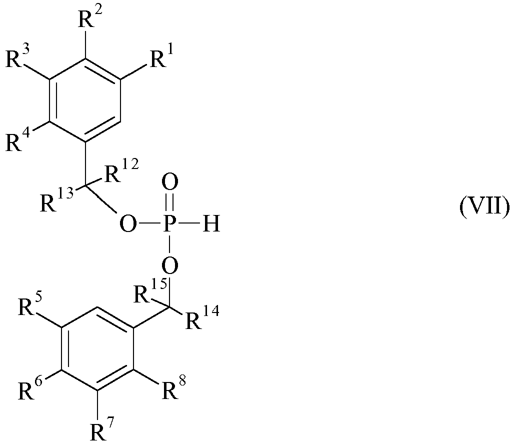
c₂) mise en réaction du composé obtenu sous b) de formule VII avec i) un composé répondant à la formule générale V 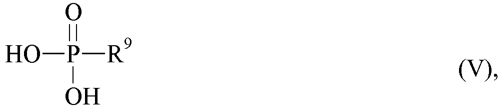 ou avec ii) de l'acide phosphorique ou un sel d'acide phosphorique, et avec R⁹ ou un composé comprenant R⁹,
R⁹ correspondant à la définition indiquée ci-dessus.

8. Procédé selon la revendication 7,
a) R¹, R³, R⁵ et R⁷
i. étant, indépendamment l'un de l'autre, H, halogène, NO₂, CN, SO₃H, un radical cyclique, acyclique, linéaire ou ramifié, substitué ou non-substitué, de nature C₁₋₂₀-aliphatique ou C₁₋₂₀-hétéroaliphatique, ou un radical, substitué ou non-substitué, de nature C₅₋₂₀-aromatique ou C₃₋₂₀-hétéroaromatique, ou
ii. étant, indépendamment l'un de l'autre, H, halogène, NO₂, CN, SO₃H, un radical cyclique, acyclique, linéaire ou ramifié, substitué ou non-substitué, de nature C₁₋₁₀-aliphatique ou C₁₋₁₀-hétéroaliphatique, ou un radical, substitué ou non-substitué, de nature C₅₋₁₂-aromatique ou C₃₋₁₂-hétéroaromatique, ou
iii. étant choisis, indépendamment l'un de l'autre, dans le groupe constitué de H, halogène, NO₂, CN, SO₃H, C₁₋₂₀-alkyle substitué ou non-substitué, C₂₋₂₀-alcényle substitué ou non-substitué, C₂₋₂₀-alcynyle substitué ou non-substitué, C₄₋₂₀-alcéninyle substitué ou non-substitué, C₃₋₂₀-cycloalkyle substitué ou non-substitué, C₃₋₂₀-cycloalcényle substitué ou non-substitué, C₅₋₂₀-cycloalcynyle substitué ou non-substitué, C₅₋₂₀-cycloalcéninyle substitué ou non-substitué, C₁₋₂₀-hétéroalkyle substitué ou non-substitué, C₂₋₂₀-hétéroalcényle substitué ou non-substitué, C₂₋₂₀-hétéroalcynyle substitué ou non-substitué, C₄₋₂₀-hétéroalcéninyle, C₅₋₂₄-aryle substitué ou non-substitué, C₃₋₂₄-hétéroaryle substitué ou non-substitué, ou
iv. étant choisis, indépendamment l'un de l'autre, dans le groupe constitué de H, halogène, NO₂, CN, SO₃H, C₁₋₁₀-alkyle substitué ou non-substitué, C₂₋₁₀-alcényle substitué ou non-substitué, C₂₋₁₀-alcynyle substitué ou non-substitué, C₄₋₁₀-alcéninyle substitué ou non-substitué, C₃₋₁₀-cycloalkyle substitué ou non-substitué, C₃₋₁₀-cycloalcényle substitué ou non-substitué, C₅₋₁₀-cycloalcynyle substitué ou non-substitué, C₅₋₁₀-cycloalcéninyle substitué ou non-substitué, C₁₋₁₀-hétéroalkyle substitué ou non-substitué, C₂₋₁₀-hétéroalcényle substitué ou non-substitué, C₂₋₁₀-hétéroalcynyle substitué ou non-substitué, C₄₋₁₀-hétéroalcéninyle, C₅₋₁₂-aryle substitué ou non-substitué, C₃₋₁₂-hétéroaryle substitué ou non-substitué, ou
v. étant tous H,
b) R_{A} et R_{B} étant différents et
i. étant, indépendamment l'un de l'autre, un radical cyclique, acyclique, linéaire ou ramifié, substitué ou non-substitué, de nature C₁₋₂₀-aliphatique ou C₁₋₂₀-hétéroaliphatique, ou un radical, substitué ou non-substitué, de nature C₅₋₂₀-aromatique ou C₃₋₂₀-hétéroaromatique, ou
ii. étant, indépendamment l'un de l'autre, un radical cyclique, acyclique, linéaire ou ramifié, substitué ou non-substitué, de nature C₁₋₁₀-aliphatique ou C₁₋₁₀-hétéroaliphatique, ou un radical, substitué ou non-substitué, de nature C₅₋₁₂-aromatique ou C₃₋₁₂-hétéroaromatique, ou
iii. étant choisis, indépendamment l'un de l'autre, dans le groupe constitué de C₁₋₂₀-alkyle substitué ou non-substitué, C₂₋₂₀-alcényle substitué ou non-substitué, C₂₋₂₀-alcynyle substitué ou non-substitué, C₄₋₂₀-alcéninyle substitué ou non-substitué, C₃₋₂₀-cycloalkyle substitué ou non-substitué, C₃₋₂₀-cycloalcényle substitué ou non-substitué, C₅₋₂₀-cycloalcynyle substitué ou non-substitué, C₅₋₂₀-cycloalcéninyle substitué ou non-substitué, C₁₋₂₀-hétéroalkyle substitué ou non-substitué, C₂₋₂₀-hétéroalcényle substitué ou non-substitué, C₂₋₂₀-hétéroalcynyle substitué ou non-substitué, C₄₋₂₀-hétéroalcéninyle, C₅₋₂₄-aryle substitué ou non-substitué, C₃₋₂₄-hétéroaryle substitué ou non-substitué, s'agissant préférentiellement de C₁₋₂₀-alkyle ou de C₁₋₂₀-alcényle,
c) R¹⁰
i. étant un radical cyclique, acyclique, linéaire ou ramifié, substitué ou non-substitué, de nature C₁₋₂₀-aliphatique ou C₁₋₂₀-hétéroaliphatique, ou un radical, substitué ou non-substitué, de nature C₅₋₂₀-aromatique ou C₃₋₂₀-hétéroaromatique, ou
ii. étant un radical cyclique, acyclique, linéaire ou ramifié, substitué ou non-substitué, de nature C₁₋₁₀-aliphatique ou C₁₋₁₀-hétéroaliphatique, ou un radical, substitué ou non-substitué, de nature C₅₋₁₂-aromatique ou C₃₋₁₂-hétéroaromatique, ou
iii. étant C₁₋₂₀-alkyle ou C₁₋₂₀-alcényle ou un radical de sucre,
et
d) R¹², R¹³, R¹⁴ et R¹⁵
i. étant, indépendamment l'un de l'autre, H, un radical cyclique, acyclique, linéaire ou ramifié, substitué ou non-substitué, de nature C₁₋₂₀-aliphatique ou C₁₋₂₀-hétéroaliphatique, ou un radical, substitué ou non-substitué, de nature C₅₋₂₀-aromatique ou C₃₋₂₀-hétéroaromatique, et/ou en accepteur d'électrons, ou
ii. étant, indépendamment l'un de l'autre, H, un radical cyclique, acyclique, linéaire ou ramifié, substitué ou non-substitué, de nature C₁₋₁₀-aliphatique ou C₁₋₁₀-hétéroaliphatique, ou un radical, substitué ou non-substitué, de nature C₅₋₁₂-aromatique ou C₃₋₁₂-hétéroaromatique, ou
iii. étant choisis, indépendamment l'un de l'autre, dans le groupe constitué de H, C₁₋₂₀-alkyle substitué ou non-substitué, C₂₋₂₀-alcényle substitué ou non-substitué, C₂₋₂₀-alcynyle substitué ou non-substitué, C₄₋₂₀-alcéninyle substitué ou non-substitué, C₃₋₂₀-cycloalkyle substitué ou non-substitué, C₃₋₂₀-cycloalcényle substitué ou non-substitué, C₅₋₂₀-cycloalcynyle substitué ou non-substitué, C₅₋₂₀-cycloalcéninyle substitué ou non-substitué, C₁₋₂₀-hétéroalkyle substitué ou non-substitué, C₂₋₂₀-hétéroalcényle substitué ou non-substitué, C₂₋₂₀-hétéroalcynyle substitué ou non-substitué, C₄₋₂₀-hétéroalcéninyle, C₅₋₂₄-aryle substitué ou non-substitué, C₃₋₂₄-hétéroaryle substitué ou non-substitué, ou
iv. étant choisis, indépendamment l'un de l'autre, dans le groupe constitué de H, C₁₋₁₀-alkyle substitué ou non-substitué, C₂₋₁₀-alcényle substitué ou non-substitué, C₂₋₁₀-alcynyle substitué ou non-substitué, C₄₋₁₀-alcéninyle substitué ou non-substitué, C₃₋₁₀-cycloalkyle substitué ou non-substitué, C₃₋₁₀-cycloalcényle substitué ou non-substitué, C₅₋₁₀-cycloalcynyle substitué ou non-substitué, C₅₋₁₀-cycloalcéninyle substitué ou non-substitué, C₁₋₁₀-hétéroalkyle substitué ou non-substitué, C₂₋₁₀-hétéroalcényle substitué ou non-substitué, C₂₋₁₀-hétéroalcynyle substitué ou non-substitué, C₄₋₁₀-hétéroalcéninyle, C₅₋₁₂-aryle substitué ou non-substitué, C₃₋₁₂-hétéroaryle substitué ou non-substitué, ou
v. étant tous H, ou
vi. étant un accepteur d'électrons ou H, à condition que R¹² et R¹⁴ représentent chacun H, et R¹³ et R¹⁵ représentent chacun un accepteur d'électrons, ou que R¹³ et R¹⁵ représentent chacun H, et R¹² et R¹⁴ représentent chacun un accepteur d'électrons.
